(19) Europäisches Patentamt
European Patent Office
Office européen des brevets

(11) EP 2 010 913 B1

(12) **FASCICULE DE BREVET EUROPEEN**

(45) Date de publication et mention de la délivrance du brevet:
**20.12.2017 Bulletin 2017/51**

(21) Numéro de dépôt: **07727947.9**

(22) Date de dépôt: **10.04.2007**

(51) Int Cl.:
***G01N 33/542*** *(2006.01)* ***G01N 33/58*** *(2006.01)*
***C12Q 1/68*** *(2006.01)*

(86) Numéro de dépôt international:
**PCT/EP2007/053479**

(87) Numéro de publication internationale:
**WO 2007/116069 (18.10.2007 Gazette 2007/42)**

(54) **PROCEDE DE SUPPRESSION D'UN SIGNAL DE FRET, AGENTS SUPPRESSEURS D'UN SIGNAL DE FRET ET UTILISATION DANS UN PROCEDE DE MULTIPLEXAGE D'EVENEMENTS BIOLOGIQUES**

VERFAHREN ZUR UNTERDRÜCKUNG EINES FRET-SIGNALS, MITTEL ZUR UNTERDRÜCKUNG EINES FRET-SIGNALS UND VERWENDUNG IN EINEM VERFAHREN FÜR BIOLOGISCHE MULTIPLEX-EREIGNISSE

METHOD FOR SUPPRESSING A FRET SIGNAL, FRET SIGNAL SUPPRESSOR AGENTS AND USE IN A METHOD FOR MULTIPLEXING BIOLOGICAL EVENTS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **10.04.2006 FR 0651294**

(43) Date de publication de la demande:
**07.01.2009 Bulletin 2009/02**

(73) Titulaire: **CISBIO BIOASSAYS**
**30200 Codolet (FR)**

(72) Inventeurs:
- **MATHIS, Gérard**
  **30200 Bagnols Sur Ceze (FR)**
- **TRINQUET, Eric**
  **30130 Pont Saint Esprit (FR)**
- **ALBERTI, Patrizia**
  **75013 Paris (FR)**
- **LAGET, Michel**
  **30130 Carsan (FR)**

(74) Mandataire: **Nevant, Marc et al**
**Cabinet Beau de Loménie**
**158, rue de l'Université**
**75340 Paris Cedex 07 (FR)**

(56) Documents cités:
**FR-A- 2 792 072** **FR-A1- 2 768 817**
**US-A1- 2002 072 625** **US-A1- 2005 118 619**
**US-A1- 2006 029 938** **US-B1- 6 472 156**

- **TRINQUET E. ET AL.: "D-myo-inositol 1-phosphate as a surrogate of D-myo-inositol 1,4,5-tris phosphate to monitor G protein-coupled receptor activation." ANAL. BIOCHEM., vol. 358, no. 1, 1 novembre 2006 (2006-11-01), pages 126-135, XP005694531 ISSN: 0003-2697**
- **BAZIN H. ET AL.: "Homogeneous time resolved fluorescence resonance energy transfer using rare earth cryptates as a tool for probing molecular interactions in biology." SPECTROCHIMICA ACTA, vol. 57, no. a, 14 septembre 2001 (2001-09-14), pages 2197-2211, XP002410783**
- **BAZIN H. ET AL.: "Time resolved amplification of cryptate emission : a versatile technology to trace biomolecular interactions." REV. MOL. BIOTECH., vol. 82, 2002, pages 233-250, XP002385176**
- **MAUREL D. ET AL.: "Cell surface detection of membrane protein interaction with homogeneous time-resolved fluorescence resonance energy transfer technology" ANAL. BIOCHEM., vol. 329, no. 2, 30 avril 2004 (2004-04-30), pages 253-262, XP002371706 ISSN: 0003-2697**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 010 913 B1

- **KENNEDY M.E. ET AL.: "Measuring human beta-secretase (BACE1) activity using homogeneous time-resolved fluorescence" ANAL. BIOCHEM., vol. 319, no. 1, 1 août 2003 (2003-08-01), pages 49-55, XP004434046 ISSN: 0003-2697**
- **LOPEZ-CRAPEZ E. ET AL.: "A separation-free assay for the detection of mutations: combination of homogeneous time-resolved fluorescence and minisequencing." HUM. MUTAT., vol. 25, mai 2005 (2005-05), pages 468-475, XP002410785**
- **TRINQUET E. ET AL.: "Fluorescence technologies for the investigation of chemical libraries." MOL. BIOSYST., vol. 2, août 2006 (2006-08), pages 381-387, XP002410786**

## Description

Les outils permettant de mesurer plusieurs événements ou molécules dans un échantillon biologique unique sont particulièrement précieux. De tels outils, en permettant le multiplexage de dosages biologiques par exemple, offrent de nouvelles perspectives dans l'étude d'évènements biologiques complexes, mais aussi dans la recherche de nouveaux médicaments.

La compréhension de certains processus biologiques ainsi que le diagnostic précis de certaines maladies peuvent nécessiter la détection simultanée de plusieurs biomolécules ou/et événements biologiques. Les technologies de multiplexage d'essais biologiques revêtent donc un intérêt remarquable dans plusieurs domaines : la recherche fondamentale, le diagnostic des maladies et également le criblage à haut débit de molécules potentiellement actives en pharmacologie. Par multiplexage, on entend la possibilité de détecter plusieurs biomolécules et/ou événements biologiques dans un échantillon biologique unique, contenus ou se produisant dans un volume réactionnel unique.

Les composés photoluminescents, c'est-à-dire capables d'absorber et de re-émettre de la lumière, sont bien adaptés pour le multiplexage d'essais biologiques.

Les techniques de multiplexage actuelles sont de deux types :

1) La plupart utilisent plusieurs composés photoluminescents pouvant être discriminés par leurs propriétés photophysiques :

- spectre d'absorption (ou d'excitation)
- et/ou spectre d'émission
- et/ou durée de vie de luminescence.
  Ces techniques nécessitent l'utilisation d'instruments sophistiqués capables:
  - d'exciter l'échantillon à plusieurs longueurs d'onde
  - et/ou de détecter plusieurs longueurs d'onde d'émission
  - et/ou de discriminer plusieurs durées de vie de luminescence.

2) D'autres techniques, utilisant des composés luminescents, reposent sur une localisation spatiale des événements biologiques à détecter. Parmi les techniques existantes permettant le mutliplexage d'événements biologiques, on peut citer :

- La technologie connue sous le nom commercial « DELFIA », commercialisée par la société PerkinElmer qui est une méthode de dosage hétérogène par fluorescence en temps résolu, comprenant une étape d'augmentation du signal.
  Une mise en oeuvre particulière de DELFIA permet d'effectuer des dosages par multidétection en phase hétérogène, fondés sur l'utilisation de chélates de lanthanides caractérisés par des temps de vie et des spectres d'émission différents. Cette technique requiert la détection de plusieurs longueurs d'onde d'émission et la discrimination temporelle des signaux. (Kimura et al. Forensic Sci Int 2000 Sep 113:345 - 51)
- La technologie connue sous le nom commercial « Trupoint », exploitée par la société PerkinElmer, qui est une méthode basée sur l'utilisation de chélates de lanthanides d'une part, d'agents extincteurs de fluorescence d'autre part («quenchers » en langue anglaise), et sur la mesure de fluorescence en temps résolu. Une mise en oeuvre particulière de cette technique, consistant à utiliser plusieurs terres rares émettant à différentes longueurs d'onde, permet d'effectuer des dosages en multidétection. Cette technique requiert la mesure de fluorescence à plusieurs longueurs d'onde d'émission et la discrimination temporelle des signaux.
- Un certain nombre de techniques basées sur des réactions en chaine de polymérisation d'acide nucléique (connues sous le terme de « PCR ») permettent la détection quantitative d'acides nucléiques.
- Les techniques connues sous les noms commerciaux de « TaqMan », « Molecular Beacon », « Scorpion », « Invader », « LightCycler », et « Amplifluor » mettent en oeuvre des fluorophores et peuvent être adaptées à la multidétection, en utilisant plusieurs fluorophores caractérisés par des spectres d'émission différents. Ces techniques requièrent la détection de la fluorescence émise par chacun de ces fluorophores, à différentes longueurs d'onde d'émission.
- Certains auteurs ont décrit une technique de multidétection en phase homogène fondée sur la détection de la polarisation de fluorescence de plusieurs traceurs à différentes longueurs d'onde d'émission (Lynch et al., 1999 ; Blommel et al., 2004).
- Dans le domaine de la microscopie, il est courant d'utiliser plusieurs composés fluorescents simultanément dans les marquages cellulaires. Ces techniques requièrent l'utilisation combinée de plusieurs longueurs d'onde d'excitation et d'émission. Les systèmes de microscopie confocale nécessitent, en plus, plusieurs sources

d'excitation.

- La technologie exploitée sous le nom commercial « FMAT » (Fluorometric Microvolume Assay technology), développée par les sociétés Beckton Dickinson et PerkinElmer Biosystems, est basée sur l'utilisation d'un LASER qui scanne des plaques micropuits. Cette technique permet la mesure d'événements biologiques par multiplexage, mais requiert l'utilisation combinée de plusieurs longueurs d'onde d'excitation et d'émission en complément de la localisation spatiale du signal via le LASER (Swartzman et al., 1999)
- La technologie connue sous le nom commercial « xMAP » de la société Luminex, est basée sur l'utilisation de microsphères colorées séparées par cytométrie de flux. Le multiplexage de la technologie repose sur l'utilisation de plusieurs sources d'excitation et de détection. L'essai ne se déroule pas vraiment en phase homogène : il y a aspiration du contenu de chaque puits pour passage dans le système en cytométrie de flux.
- La société Meso Scale Discovery a développé une technologie, en phase homogène, qui permet le multiplexage d'essais biologiques en utilisant un seul composé luminescent. Cette technologie repose sur la localisation spatiale de chaque analyte à détecter : le volume réactionnel est déposé dans un puits d'une microplaque, les différents analytes sont capturés et détectés sur des points distincts pre-déposés sur le fond du puits. Les technologies développées par les sociétés Luminex et Meso Scale Discovery permettent un degré élevé de multiplexage ; en revanche elles ne sont pas adaptées pour la détection de biomolécules à l'intérieur de cellules intactes ou en milieu homogène, un support solide étant requis.
- La technologie connue sous le nom commercial « eTag™ » de la société Aclara, est une technologie permettant le multiplexage d'essais biologiques en utilisant une seule longueur d'onde d'excitation et une seule longueur d'onde d'émission. Néanmoins elle requiert la discrimination de chaque sonde par électrophorèse capillaire. Elle repose en fait sur l'utilisation de sondes caractérisées par les mêmes propriétés de fluorescence, mais possédant différents rapports masse/charge (Chan-Hui et al. 2004 ; Tian et al. 2004).
- Bradford et al. (2004) ont décrit un système de multidétection combinant cytométrie de flux et intensité de fluorescence: l'utilisation du système de marquage indirect développé par Molecular Probes (ZENON technology) permet d'obtenir des conjugués fluorescents possédant chacun une intensité de fluorescence définie. Chaque conjugué anticorps-composé fluorescent est dirigé contre un antigène de surface cellulaire donné. En détectant la fluorescence dans un cytomètre de flux, on peut connaître le pourcentage de cellules exprimant tel ou tel antigène de surface en fonction de l'intensité de fluorescence mesurée sur les cellules.

Quelques articles additionnels présentent des composés fluorescents pouvant permettre la mise en oeuvre d'essais multiplexés :

- Kurner et al., 2001 décrivent des nanosphères phosphorescentes (commercialisées par la société Chromeon) caractérisées par des temps de vie et des longueurs d'onde d'émission différentes qui peuvent être excitées à la même longueur d'onde.
- Tong et al. (2001) décrivent l'utilisation de 3 fluorophores pour la fabrication de 8 sondes, excitables à la même longueur d'onde ; 3 longueurs d'onde de détection sont nécessaires pour l'identification des sondes. (technique connue sous le terme « CFET tags », pour « Combinatorial fluorescence energy transfer tags » en langue anglaise)
- Tyagi et al. (2000) décrivent des balises moléculaires constituées d'acides nucléiques marqués par des fluorophores (connues sous le terme anglo-saxon de « molecular beacons ») excitables à la même longueur d'onde et émettant à des longueurs d'onde différentes.

Les techniques de l'art antérieur sont donc toutes basées sur des appareillages de mesure permettant de discriminer les signaux émis par le milieu réactionnel : appareillages permettant la mesure de signaux lumineux émis à différentes longueurs d'ondes, appareillages permettant de séparer les signaux par électrophorèses (etags), dispositifs permettant une localisation spatiale spécifique de chaque évènement biologique étudié, et donc de chaque signal.

La mise en oeuvre de ces techniques pour le multiplexage d'événements biologiques n'est donc pas toujours aisée puisqu'elles nécessitent le plus souvent un appareillage complexe, ou bien des étapes de séparation ou de lavage, qui ne sont pas toujours adaptées aux besoins de l'industrie pharmaceutique, notamment les besoins dans le domaine du criblage à haut débit.

Un certain nombre de composés possédant un domaine de liaison à des fluorophores ont été décrits, ainsi que leurs effets éventuels sur les propriétés photophysiques des fluorophores qu'ils reconnaissent.

Le brevet américain US 6,747,135 divulgue des polypeptides, appelés « fluorettes » ayant une affinité élevée pour des fluorophores. Ces fluorettes sont particulièrement utiles pour coupler des fluorophores à des protéines, de manière non-covalente. Par ailleurs ce brevet divulgue une fluorette spécifique du rouge texas, qui provoque un décalage des spectres d'absorption et d'émission de ce fluorophore. Les autres fluorettes présentées n'ont pas d'effet significatif sur les propriétés de fluorescence.

Watt et Al (1977) décrivent que la liaison d'un anticorps anti-fluorescéine avec la fluorescéine modifie les

propriétés photophysiques de cette dernière: baisse de 90% de son rendement quantique, déplacement de ses longueurs d'ondes maximum d'émission et d'excitation.

La demande de brevet US 2005 /0064512 divulgue des polypeptides, en particulier des anticorps, liant spécifiquement des fluorophores organiques, notamment de la famille des cyanines. Les anticorps selon US 2005/0064512 provoquent des changements dans les propriétés photophysiques de ces fluorophores et notamment un changement important dans leur maximum d'excitation et d'émission.

Babendure et al., 2003, décrivent de courtes séquences nucléotidiques (appelées Aptamères) capables de se lier à des fluorophores organiques tels que ceux comportant un motif triphénylméthane. Les auteurs ont par ailleurs mis en évidence une augmentation de la fluorescence émise lors de la liaison de l'aptamère au fluorophore.

La société Molecular Probes commercialise un certain nombre d'anticorps, dont la plupart sont des anticorps polyclonaux, ayant une affinité pour des fluorophores tels que des anticorps anti-fluorescéine, des anticorps anti Alexa Fluor 488, des anticorps anti-tétraméthylrhodamine, des anticorps anti-Bodipy, des anticorps anti-dansyl. Ces anticorps modifient les propriétés des fluorophores auxquels ils se lient : ils entraînent une extinction ou au contraire une exaltation de fluorescence.

Ces composés possèdent tous un domaine de liaison à un fluorophore et certains d'entre eux entraînent des variations des propriétés spectroscopiques lorsqu'ils se lient au fluorophore, mais aucun d'eux n'a été décrit en tant qu'agent suppresseur de FRET. De plus, on peut noter que leurs effets ne sont pas homogènes, puisque certains vont décaler les spectres d'émission ou d'excitation, d'autres vont provoquer une exaltation ou une extinction de fluorescence, et d'autres enfin ne vont pas modifier les caractéristiques des fluorophores.

## DESCRIPTION DE L'INVENTION

La demanderesse a découvert une méthode permettant de supprimer un signal de FRET (« Fluorescence Resonance Energy Transfer », ou fluorescence résultant d'un transfert d'énergie de résonance). Cette méthode a permis le développement de procédés de multiplexage d'évènements biologiques dans un milieu réactionnel. Certains moyens mis en oeuvre dans ces procédés sont également l'objet de la présente invention, en particulier certains agents suppresseurs du signal FRET, ainsi que des nécessaires de composants (« kits of parts » en langue anglaise) permettant la mise en oeuvre des procédés selon l'invention.

Les procédés de multiplexage selon l'invention présentent de nombreux avantages par rapport aux procédés de l'art antérieur, puisqu'ils peuvent être mis en oeuvre en milieu homogène, ne nécessitent aucune étape de séparation, de lavage, et peuvent être utilisés avec les fluorimètres habituellement utilisés pour détecter un événement biologique unique. De plus, ces procédés ne nécessitent pas de résolution temporelle des signaux ni de localisation spatiale des biomolécules à détecter.

Les procédés selon l'invention sont basés sur la technique de FRET qui consiste à mesurer le transfert d'énergie non radiatif de résonance entre un fluorophore donneur et un fluorophore accepteur. Après excitation lumineuse à la longueur d'onde d'excitation du fluorophore donneur, un transfert d'énergie a lieu entre le fluorophore donneur et le fluorophore accepteur s'ils sont proches l'un de l'autre.

La détection du phénomène de FRET peut se faire par la mesure de différents paramètres du signal de fluorescence émis soit par le donneur, soit par l'accepteur, soit par les deux molécules. Parmi les techniques les plus utilisées, on peut notamment citer :

- La mesure de la baisse de fluorescence du donneur induite par le phénomène de FRET ;
- La mesure de l'augmentation de la fluorescence de l'accepteur induite par l'énergie provenant du donneur au travers du FRET ;
- La détermination du ratio [(augmentation fluorescence accepteur)/ (diminution fluorescence donneur)] ;
- La mesure de la diminution de la durée de vie de fluorescence du donneur induite par le phénomène de FRET. Celle-ci est notamment mesurée par la technique de « Fluorescence Lifetime Imaging Microscopy » (FLIM) ;
- La mesure de l'augmentation de la fluorescence du donneur impliqué dans un FRET après le photoblanchiment de l'accepteur. Cette technique de photoblanchiment est connue sous le nom de Fluorescence Recovery After Photobleaching (FRAP).

La technique de FRET est une technique de choix pour étudier des interactions chimiques ou biologiques qui entraînent une modification de la distance entre un fluorophore donneur et un fluorophore accepteur : le principe général consiste à préparer des conjugués fluorescents en couplant les partenaires de FRET à des molécules impliquées dans un processus biologique ou à des sondes reconnaissant de telles molécules, et à mesurer les variations de FRET en réponse à une stimulation, par exemple en ajoutant dans le milieu des composés qui vont affecter le processus biologique étudié. Ces composés peuvent par exemple intervenir dans la régulation de réactions enzymatiques, provoquer des modifications de conformation tridimensionnelle de protéines, provoquer la production d'un analyte et la formation d'un

complexe analyte/partenaires de FRET ; dans tous les cas, une modification de l'évènement biologique étudié provoque une modification du FRET entre les composés fluorescents donneurs et accepteurs.

## I- Procédé de suppression de FRET

La demanderesse a maintenant mis au point un procédé basé sur la suppression du FRET entre deux conjugués fluorescents partenaires de FRET et spécifiques d'un événement biologique, non pas en intervenant sur cet événement biologique, mais sur les partenaires de FRET.

Ce procédé de suppression du FRET émis par un milieu réactionnel contenant un couple de conjugués fluorescents partenaires de FRET spécifiques d'un événement biologique est caractérisé en ce que l'on introduit dans ce milieu au moins un agent suppresseur du signal de FRET, ledit agent suppresseur du signal de FRET ne perturbant pas ledit événement biologique.

Ce procédé peut être utilisé dans le cadre de l'étude de nombreux évènements biologiques tels que : les évènements biologiques impliquant des réactions enzymatiques (telle que les activités de type kinase, phosphorylase, transférase, hydrolase, lyase, ligase, isomérase, polymérase...), la présence ou la variation de concentration d'un analyte dans le milieu réactionnel (par exemple la présence ou variation de concentration de protéines, enzymes, substrats d'enzymes, messagers intracellulaires, tels que les nucléotides cycliques (AMPc, GMPc etc...), les phospholipides (PIP2, PIP3...), les facteurs de transcription, les composés comportant un cycle inositol (IP1, IP2, IP3, IP4...), les acides nucléiques, les facteurs de croissance, le rapprochement de deux molécules dans le milieu réactionnel, les modifications de conformation tridimensionnelle de molécules. Les exemples décrits illustrent quelques applications de l'invention mais il serait injustifié de limiter l'invention à ces évènements.

La demanderesse a mis en évidence, de manière surprenante, que certains composés peuvent supprimer le signal de FRET résultant du rapprochement de 2 conjugués partenaires de FRET.

Par agent suppresseur du signal de FRET, on entend un composé tel que défini ci-après, qui provoque une diminution de signal de FRET d'au moins 70% par rapport au signal mesuré en l'absence de ce composé. Les agents suppresseurs du signal de FRET provoquant une diminution du FRET d'au moins 80%, ou d'au moins 90% sont préférés.

Les agents suppresseurs du signal de FRET utilisés dans le procédé selon l'invention ne perturbent pas l'événement biologique étudié dans la mesure où ils agissent au niveau d'un des fluorophores impliqués dans le FRET, et non pas au niveau de l'évènement biologique étudié : ils n'ont aucun effet sur la distance séparant les molécules impliquées dans l'événement biologique.

Afin de déterminer si un composé est suppresseur du signal de FRET au sens de l'invention, il peut être mis en contact avec un couple de conjugués fluorescents partenaires de FRET émettant un signal de FRET, par exemple deux conjugués fluorescents reconnaissant une même molécule, ou encore deux conjugués fluorescents dont l'un comprend une biotine et l'autre la streptavidine ; Le signal de FRET émis par le milieu réactionnel est mesuré en présence et en l'absence dudit composé, les suppresseurs du signal FRET étant ceux qui entraînent une diminution de signal d'au moins 70%. Ce test simple permet à l'homme du métier d'isoler des agents suppresseurs de FRET au sens de l'invention.

Ces agents peuvent être de différente nature, avoir différentes fonctions, mais dans tous les cas permettent de supprimer le signal de FRET émis par un couple donneur-accepteur donné :

a) Agents suppresseurs de signal de FRET agissant par fixation spécifique par liaison non covalente sur l'un des fluorophores impliqués dans le FRET.

Ces agents possèdent un domaine permettant une liaison spécifique et non-covalente avec le fluorophore.

Dans le cas d'un FRET impliquant un chélate ou un cryptate de terre rare, les agents suppresseurs du signal FRET sont les anticorps anti-chélate de terre rare ou anti-cryptate de terre rare.

Les anticorps anti-cryptates sont produits par les techniques bien connues de l'homme du métier d'immunisation d'un mammifère avec un antigène.

Dans le cas où l'antigène est de petite taille, comme c'est le cas du cryptate de terre rare, il doit être couplé à une molécule porteuse immunogène. Pour cela, le cryptate est fonctionnalisé, comme décrit dans le brevet EP 321 353. De préférence, un bras alkylamine est greffé au cryptate.

Les molécules porteuses peuvent être choisies parmi : la sérum albumine bovine (BSA) ou la BSA cationique (cBSA), la KLH (keyhole Limpets Hemocyanine ou hémocyanine de patelle), la thyroglobuline, l'ovalbumine. Les molécules porteuses peuvent également être des liposomes ou des molécules porteuses synthétiques comme des polymères de L-Lysine ou d'acide L-glutamique, du ficoll, du dextran, ou encore du polyéthylène glycol. Ces molécules porteuses comportent généralement des groupes fonctionnels qui réagiront avec le cryptate fonctionnalisé, ou bien de tels groupes pourront être introduits par des techniques classiques. De préférence, un cryptate de terre rare portant un groupe alkylamine est conjugué à la BSA.

L'immunogène ainsi obtenu est ensuite mélangé à un adjuvant, comme par exemple l'adjuvant de Freund.

Des mammifères - par exemple des souris - sont immunisés par injection sous-cutanée de cette solution, et après une période nécessaire à l'induction de l'immunité, les sérums des animaux sont recueillis et les anticorps polyclonaux purifiés, par exemple par chromatographie d'affinité.

Les anticorps monoclonaux anti-cryptate sont produits en utilisant diverses techniques connues de l'homme du métier. A titre d'exemple, on peut évoquer la technique issue des travaux de Köhler et Milstein : quelques semaines après l'immunisation, la rate de la souris immunisée avec l'antigène est prélevée. Un mélange de lymphocytes et de plasmocytes provenant de cette rate est fusionné in vitro avec des cellules myélomateuses en présence d'un inducteur de fusion cellulaire, comme le polyéthylène glycol. Une lignée cellulaire myélomateuse mutante, dépourvue d'hypoxanthine guanosine phosphoribosyl transférase (HGPRT), est utilisée pour permettre de sélectionner aisément les cellules hybrides. Ces cellules sont cultivées dans un milieu contenant de l'hypoxanthine, de l'aminoptérine (méthotréxate) et de la thymine (milieu HAT) afin d'éliminer les cellules myélomateuses non fusionnées et de sélectionner les hybridomes d'intérêt. Les cellules de rate non fusionnées meurent car elles ne sont pas capables de proliférer in vitro. Les cellules hybrides, en revanche, survivent. Les hybridomes ainsi obtenus sont cultivés dans les puits d'une plaque de culture cellulaire. Les surnageants de ces puits sont testés pour la présence d'anticorps spécifiques anti-cryptate de terre rare dans un test de criblage simple tel que l'ELISA ou RIA. Les hybridomes sont ensuite clonés et peuvent être injectés à des mammifères pour induire des myélomes sécrétant en grande quantité des anticorps anti-cryptate dans le liquide d'ascite.

Les anticorps anti-cryptate de terre rare poly- ou monoclonaux obtenus, peuvent être ensuite testés pour leur capacité à supprimer le signal de FRET émis par un couple de partenaires de FRET comprenant ledit cryptate de terre rare en tant que donneur d'énergie. Pour cela, on peut procéder comme décrit ci-dessus et dans l'exemple 1 présenté ci-après.

b) <u>Agents suppresseurs de FRET agissant par découplage du fluorophore avec l'événement biologique.</u>

Comme décrit plus haut, l'étude d'événements biologiques en utilisant le phénomène de FRET est basée sur le marquage de molécules impliquées dans l'événement biologique par des fluorophores partenaires de FRET. Si ce marquage est effectué de manière non covalente, via des partenaires de liaison, il est possible de découpler un des fluorophores de l'événement biologique.

Par exemple, si une molécule impliquée dans l'événement biologique est lié de manière covalente à un membre d'un couple de partenaires de liaison (par exemple un oligonucléotide simple brin), et que le fluorophore est lié de manière covalente à l'autre membre de ce couple (par exemple un oligonucléotide simple brin complémentaire), alors l'ajout dans le milieu de mesure du premier membre de ce couple (le premier oligonucléotide simple brin, sous forme libre) va découpler le marquage de la molécule biologique par le fluorophore, par un phénomène de compétition selon le schéma représenté sur la figure 1.

Ces agents suppresseurs de signal de FRET sont des oligonucléotides formant un complexe non-covalent avec l'un des conjugués fluorescents partenaires de FRET.

c) <u>Agents suppresseurs de FRET agissant par modification de la nature du fluorophore</u>

Dans le cas où le fluorophore est un chélate de terre rare, les agents suppresseurs de FRET peuvent réduire la stabilité du fluorophore, en rentrant en compétition soit avec la terre rare pour la liaison avec le chélate, soit avec le chélate pour la liaison avec la terre rare.

Par exemple, l'ajout au milieu de mesure d'un ion qui va rentrer en compétition avec la terre rare pour la liaison avec le chélate peut favoriser la formation d'un nouveau complexe non-fluorescent chélate-ion. Un tel ion peut être l'ion Manganèse $Mn^{2+}$ dans le cas des chélates de terre rare.

A titre d'exemple de compétition avec le chélate, on peut citer l'utilisation d'agents complexant les métaux, tels que l'EDTA, qui va entraîner la formation d'un complexe EDTA-terre rare, non fluorescent, au détriment du complexe fluorescent chélate-terre rare.

d) <u>Agents suppresseurs de FRET agissant par modification des propriétés photophysiques du fluorophore</u>

Ce type d'agents suppresseurs de FRET entraîne un effet d'extinction de fluorescence (« quenching » en langue anglaise).

On peut citer à titre d'exemple l'acide urique qui, dans le cas où le fluorophore est un chélate ou un cryptate de terre rare, peut provoquer des réactions d'oxydo-réduction de la terre rare qui peuvent aboutir à une suppression du FRET.

Couple de conjugués fluorescents partenaires de FRET.

Un couple de conjugués fluorescents partenaires de FRET au sens de l'invention est constitué d'un conjugué fluorescent donneur et d'un conjugué fluorescent accepteur dont les spectres d'émission et d'absorption sont compatibles. Chacun de ces conjugués est constitué d'un fluorophore lié de manière covalente à une sonde, cette sonde étant impliquée dans un événement biologique, ou bien reconnaissant de manière spécifique une molécule impliquée dans un événement biologique.

La notion de partenaires de FRET est bien connue de l'homme du métier qui est capable, sur la base des caractéristiques spectroscopiques des composés fluorescents connus, de sélectionner des couples de fluorophores compatibles en termes de FRET. Il pourra en outre se référer à l'ouvrage de Lakowicz, Principles of fluorescence spectroscopy, 2nd edition, Kluwer academic/plenum publishers, NY (1999). Par ailleurs, le terme FRET est ici utilisé au sens large et inclus le FRET en temps résolu (TR-FRET).

*Les fluorophores :*

Les fluorophores peuvent être choisis parmi le groupe suivant comprenant : les protéines luminescentes, telles que la protéine fluorescente verte (GFP) ou ses variants, les protéines fluorescentes extraites de coraux, les phycobiliprotéines, telles que la B-phycoérythrine, la R-phycoérythrine, la C-phycocyanine, les allophycocyanines, en particulier celles connues sous la dénomination XL665 ; les molécules organiques luminescentes, telles que les rhodamines, les cyanines, les squaraines, les fluorophores connus sous la dénomination BODIPY, les fluorescéines, les composés connus sous la dénomination AlexaFluor; les complexes supra-moléculaires, tels que les cryptates de terre rare, les chélates de terre rare (en particulier les chélates et cryptates d'europium, de terbium, de samarium, de dysprosium, de neodymium); les particules inorganiques luminescentes comme les quantum dots; ces fluorophores peuvent être utilisés soit comme donneurs soit comme accepteurs dans un système de FRET.

Les complexes de terre rare sont des composés connus qui sont décrits par exemple dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. D'autres chélates sont composés d'un ligand nonadenté tel que la terpyridine (EP 403 593, US 5 324 825, US 5 202 423, US 5 316 909). La terre rare peut être l'europium ou le terbium.

Les cryptates de terres rares sont décrits dans les brevets EP 0 180 492, EP 0 601 113 et la demande WO 01/96 877.

Avantageusement, le complexe de terre rare est un chélate ou un cryptate d'europium. Lorsque la terre rare est l'europium, le complexe de terre rare est de préférence un cryptate de terre rare à motif pyridine, de manière encore plus préférée à motif pyridine-bipyridine.

*Les* sondes :

La sonde présente dans un conjugué fluorescent peut être une molécule directement impliquée dans l'événement biologique étudié, ou encore une molécule reconnaissant une autre molécule impliquée dans ledit évènement, par exemple un anticorps anti-protéine d'intérêt, dans le cas ou l'événement biologique est une interaction entre deux protéines d'intérêt.

De manière générale et selon l'invention, la notion de molécule impliquée dans un évènement biologique signifie que cette molécule joue un rôle dans l'événement biologique, ou bien est recrutée ou à tendance à s'accumuler sur le site de l'événement biologique. Par exemple, si l'événement biologique est la production d'un analyte, une molécule impliquée dans cet événement pourra être : un précurseur de l'analyte, une enzyme participant à la synthèse de cet analyte, un anticorps reconnaissant l'analyte.

Les molécules impliquées dans des évènements biologiques peuvent être : des protéines, des peptides, des enzymes, des substrats d'enzymes, des messagers intracellulaires tels que les nucléotides cycliques (AMPc, GMPc etc.), des phospholipides (PIP2, PIP3...), des facteurs de transcription, des composés comportant un cycle inositol (IP1, IP2, IP3, IP4...), des acides nucléiques, des oligonucléotides, des facteurs de croissance.

Les composés reconnaissant des molécules impliquées dans des évènements biologiques peuvent être : des anticorps ou des fragments d'anticorps, des anticorps ou fragments d'anticorps anti-étiquette (anti-tag) reconnaissant des étiquettes (tags) greffées sur les molécules impliquées dans les évènements biologiques, des aptamères, des peptides, des protéines, des acides nucléiques simple-brin pouvant s'hybrider avec une séquence complémentaire greffée sur les molécules impliquées dans des évènements biologiques . Les systèmes tag/anti-tag couramment utilisés incluent les couples : DNP/anticorps anti-DNP, dans lequel DNP représente le dinitrophénol ; GST/anticorps anti-GST dans lequel GST représente la glutatione S-transférase ; biotine/avidine ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG®/anticorps anti-FLAG ® dans lequel FLAG ® est un peptide ayant

les 8 acides aminés ci-après DYKDDDDK ; HA/anticorps anti HA dans lequel HA est un épitope de l'hémaglutinine d'Influenza.

Les sondes détermineront donc la spécificité d'un couple de conjugués fluorescents partenaires de FRET pour un événement biologique donné. Le procédé selon l'invention peut être utilisé pour étudier de nombreux évènements biologiques tels que : les évènements biologiques impliquant des réactions enzymatiques (telle que les activités de type kinase, phosphorylase, transférase, hydrolase, lyases, ligases, isomérase, polymérase...), la présence ou la variation de concentration d'un analyte dans le milieu réactionnel (par exemple la présence ou variation de concentration de protéines, enzymes, substrats d'enzymes, messagers intracellulaires tels que les nucléotides cycliques (AMPc, GMPc etc.), les phospholipides (PIP2, PIP3...), les facteurs de transcription, les composés comportant un cycle inositol (IP1, IP2, IP3, IP4...), les acides nucléiques, les facteurs de croissance, le rapprochement de deux molécules dans le milieu réactionnel, les modifications de conformations tridimensionnelles de molécules. Les exemples décrits ci-après illustrent quelques applications de l'invention mais il serait injustifié de limiter l'invention à ces évènements.

L'homme du métier est à même de préparer des couples de conjugués fluorescents partenaires de FRET spécifiques d'un évènement biologique : la conjugaison d'un fluorophore avec une sonde au sens de l'invention est en effet largement décrite dans la littérature (par exemple dans « Bioconjugate Techniques », G.T. Hermanson, Academic Press, 1996.) et fait appel à l'utilisation des groupes réactionnels classiquement utilisés.

Le procédé de suppression du signal de FRET dans un milieu réactionnel est particulièrement utile lorsque plusieurs signaux de FRET issus de plusieurs couples de conjugués fluorescents partenaires de FRET, spécifiques de plusieurs évènements biologiques, doivent être mesurés : il rend en effet possible la détection d'évènements biologiques par multiplexage.

**<u>II- Procédé de multiplexage d'évènements biologiques</u>**

Un objet de l'invention est un procédé de détection de deux évènements biologiques dans un milieu réactionnel qui consiste :

1) à introduire dans ce milieu :

- 2 couples de conjugués fluorescents partenaires de FRET, au moins l'un des conjugués fluorescents comprenant un chélate ou un cryptate de terre rare, chaque couple étant spécifique d'un des évènements biologiques étudiés, la longueur d'onde à laquelle les conjugués fluorescents donneurs sont excités étant la même pour tous les couples, et la longueur d'onde à laquelle sont mesurés les signaux émis par les conjugués fluorescents accepteurs étant la mêmes pour tous les couples ;
- un agent suppresseur de signal de FRET capable de supprimer sélectivement le FRET entre deux partenaires de FRET donnés, ledit agent étant un composé qui provoque une diminution de signal de FRET d'au moins 70% par rapport au signal mesuré en l'absence de ce composé ; et

2) à mesurer de manière séquentielle les différents signaux de FRET émis par le milieu réactionnel ;
ledit agent suppresseur de signal de FRET étant tel que défini à la revendication 1. Selon l'invention, le procédé de détection de deux évènements biologiques dans un milieu réactionnel comprend les étapes suivantes :

(i) mise en contact du milieu réactionnel avec un premier couple de conjugués fluorescents partenaires de FRET spécifique d'un premier évènement biologique ;
(ii) excitation du milieu réactionnel à la longueur d'onde $\lambda 1$ ;
(iii) mesure du signal de FRET émis par le milieu réactionnel à la longueur d'onde À2 (signal correspondant au premier événement biologique) ;
(iv) ajout d'un agent suppresseur du signal de FRET spécifique du couple de partenaires de FRET mis en contact dans le milieu, ledit agent suppresseur du signal de FRET étant tel que défini à la revendication 1 ;
(v) mise en contact du milieu réactionnel avec un couple de conjugués fluorescents partenaires de FRET spécifiques du second évènement biologique;
(vi) excitation du milieu réactionnel à la longueur d'onde $\lambda 1$ ;
(vii) mesure du signal de FRET émis par le milieu réactionnel à la longueur d'onde À2 (signal correspondant à l'événement biologique dont est spécifique le second couple de partenaires de FRET mis en contact dans le milieu) ; les conjugués fluorescents partenaires de FRET comprenant un fluorophore donneur et un fluorophore accepteur, $\lambda 1$ étant la longueur d'onde d'excitation des fluorophores donneurs et À2 étant la longueur d'onde d'émission des fluorophores accepteurs.

Les conjugués fluorescents partenaires de FRET et les agents suppresseurs du signal FRET ont été définis à

la revendication 1. L'expression « mise en contact du milieu réactionnel avec un couple de conjugués fluorescents partenaires de FRET spécifiques d'un évènement biologique» signifie que les partenaires de FRET se retrouvent simultanément dans le milieu réactionnel. On peut parvenir à ce résultat en les introduisant simultanément au cours de l'une des étapes (i) ou (v) ou bien en les introduisant de manière séquentielle dans le milieu réactionnel : dans ce cas, la mise en contact correspondra à l'ajout du deuxième membre de ce couple, à condition que son partenaire ait été introduit dans une étape antérieure, par exemple au cours de l'étape (i). Selon l'invention, les couples de conjugués fluorescents partenaires de FRET sont en effet formés de manière séquentielle.

Ainsi, dans une mise en oeuvre particulière du procédé selon l'invention, l'étape (i) consiste à introduire d'une part un membre de chacun des deux couples de conjugués fluorescents partenaires de FRET spécifiques des deux évènements biologiques à étudier, (soit deux membres de deux couples) et à introduire d'autre part un second membre de l'un des ces couples pour effectivement mettre en contact le couples de conjugués fluorescents partenaires de FRET spécifique du premier évènement biologique à détecter. Les seconds membres des autres couples de conjugués fluorescents partenaires de FRET sont introduits de manière successive, dans chacune des étapes (v) suivantes.

Dans une autre mise en oeuvre, les membres des couples de conjugués fluorescents partenaires de FRET sont introduits simultanément dans le milieu de mesure, le premier couple étant introduit dans le milieu réactionnel dans l'étape (i), et l'autre couple étant introduit de manière successive dans l'étape (v). Le procédé précédent repose sur le principe de la formation séquentielle de couples de partenaires de FRET et la mesure puis l'extinction séquentielle du signal de FRET associé. Chaque signal mesuré est représentatif d'un événement biologique donné.

Un procédé alternatif consiste à introduire l'ensemble des deux couples partenaires de FRET dans le milieu de mesure, à mesurer le signal total émis et correspondant à la somme des signaux spécifiques à chaque événement biologique, puis à introduire séquentiellement les agents suppresseurs de FRET et à mesurer le signal émis après chaque étape d'introduction desdits agents. L'ensemble des mesures permet, par une méthode mathématique simple, de calculer la valeur du signal spécifique à chaque couple de partenaire de FRET et donc à chaque évènement biologique.

Ce procédé alternatif de détection de deux évènements biologiques dans un milieu réactionnel comprend les étapes suivantes :

(i) mise en contact du milieu réactionnel avec les deux couples de conjugués fluorescents partenaires de FRET spécifiques de deux évènements biologiques ;
(ii) excitation du milieu réactionnel à la longueur d'onde $\lambda 1$ ;
(iii) mesure du signal de FRET émis par le milieu réactionnel et correspondant à l'ensemble des événements biologiques étudiés ;
(iv) introduction dans le milieu réactionnel d'un agent suppresseur du signal de FRET spécifique d'un desdits couples de conjugués fluorescents partenaires de FRET, ledit agent suppresseur du signal de FRET étant tel que défini à la revendication 1 ;
(v) excitation du milieu réactionnel à la longueur d'onde $\lambda 1$ ;
(vi) mesure du signal de FRET émis par le milieu réactionnel, correspondant à tous les évènements réactionnels moins ceux dont le signal a été bloqué dans une étape précédente) ;
(vii) détermination des valeurs de signal de FRET pour chacun des deux événements biologiques, par la résolution mathématique des deux équations obtenues dans les étapes (iii) et chacune des étapes (vi) ; les conjugués fluorescents partenaires de FRET comprenant un fluorophore donneur et un fluorophore accepteur, $\lambda 1$ étant la longueur d'onde d'excitation des fluorophores donneurs et À2 étant la longueur d'onde d'émission des fluorophores accepteurs.

Comme décrit plus haut, les procédés selon l'invention font intervenir plusieurs couples de conjugués fluorescents partenaires de FRET spécifiques d'un événement biologique donné. Chaque couple est constitué de deux conjugués, chacun comprenant un fluorophore couplé de manière covalente à une sonde spécifique. Il est tout à fait possible que certains couples aient des éléments communs : deux couples donnés peuvent ainsi partager le même conjugué donneur, le même conjugué accepteur, ou encore comporter les mêmes fluorophores donneurs, ou les mêmes fluorophores accepteurs.

Certains couples partagent un conjugué fluorescent, autrement dit un des conjugués fluorescents va avoir plusieurs partenaires de FRET. Par exemple, un premier couple peut être constitué d'un membre flurophorel-sondel et d'un membre fluorophore 2-sonde 2, et l'autre couple peut être constitué d'un membre fluorophore1-sonde1 et fluorophore 3-sonde 3.

Certains couples peuvent comporter les mêmes fluorophores mais dans ce cas ils diffèrent par leurs sondes : Par exemple, un premier couple peut être constitué d'un membre fluorophore1-sonde1 et d'un membre fluorophore2-sonde 2, et l'autre couple peut être constitué d'un membre fluorophore1-sonde3 et fluorophore 3-sonde 4.

Dans les procédés selon l'invention, chaque suppresseur de signal de FRET utilisé est spécifique d'un seul couple de partenaires de FRET. Ceci est une des caractéristiques essentielles de l'invention permettant de différencier

les signaux correspondant à chaque événement biologique que l'on souhaite étudier.

Les techniques de FRET en temps résolu offrent des avantages certains pour l'étude d'événements biologiques ayant lieu dans des milieux biologiques. Ainsi, dans une mise en oeuvre particulière des procédés selon l'invention, les conjugués fluorescents partenaires de FRET sont partenaires de TR-FRET (en langue anglaise « Time Resolved FRET »).

L'étude d'évènements biologiques peut nécessiter une étape de stimulation du milieu réactionnel afin de déclencher ou faire varier les évènements biologiques que l'on souhaite étudier. Cette stimulation peut être, notamment, de nature chimique, pharmacologique, électrique, thermique ou mécanique. Par exemple, si l'événement biologique étudié est la production d'un analyte dans une cellule en réponse à la stimulation d'un récepteur transmembranaire, il sera nécessaire d'ajouter un agoniste dudit récepteur.

Dans une mise en oeuvre particulière, l'étape de stimulation consiste à rajouter au milieu réactionnel un agoniste, un antagoniste, un modulateur allostérique ou un agoniste inverse d'un récepteur donné.

Dans la mesure où les procédés selon l'invention permettent d'étudier plusieurs événements biologiques dans un même milieu réactionnel, il peut être nécessaire d'effectuer plusieurs stimulations.

Par ailleurs, une même stimulation peut activer plusieurs voies de signalisation parallèles ou encore une cascade de voies de signalisation et donc plusieurs évènements biologiques détectables grâce aux procédés selon l'invention. Dans ce cas, ces procédés ne comprendront qu'une seule étape de stimulation qui provoquera une variation des différents évènements biologiques à détecter. On peut citer l'exemple d'une stimulation pharmacologique entraînant l'activation de protéines kinases (évènement biologique 1) qui vont elles-mêmes entraîner la phosphorylation de protéines (événement 2).

Les procédés selon l'invention permettent de détecter plusieurs évènements biologiques et donc bien sûr leur variation en présence d'agents dont on veut déterminer l'effet sur plusieurs évènements biologiques ayant lieu dans le milieu réactionnel. Les procédés selon l'invention peuvent donc être avantageusement utilisés dans une méthode de criblage de composés biologiquement actifs qui peuvent être utiles, par exemple, comme médicaments. Pour cela, le procédé selon l'invention peut comprendre une étape d'ajout d'un composé à tester dans le milieu réactionnel.

Le procédé selon l'invention peut être mis en oeuvre dans divers milieux réactionnels, en particulier des milieux comprenant un mélange de composés biologiques, tels qu'un mélange de protéines, d'anticorps, d'acides nucléiques, de messagers intracellulaires, extracellulaires etc. Le milieu réactionnel peut également contenir des cellules provenant de cultures cellulaires, des cellules obtenues par dissociation de tissus, des cellules perméabilisées, des broyats cellulaires, des préparations de membranes.

Dans une mise en oeuvre avantageuse du procédé de l'invention, l'un des conjugués fluorescents comporte un chélate ou un cryptate d'europium tris-bipyridine, et l'agent suppresseur du signal de FRET est un anticorps anti-cryptate d'europium tris-bipyridine.

Dans une autre mise en oeuvre avantageuse du procédé de l'invention, au moins un des couples de conjugués fluorescents partenaires de FRET comprend un chélate de terre rare, et l'agent suppresseur du signal de FRET est l'ion $Mn^{2+}$ ou l'EDTA.

Le milieu réactionnel est par ailleurs contenu dans un contenant classiquement utilisé, par exemple un puits de microplaque, une chambre d'incubation cellulaire adaptable sur microscope.

L'homme du métier est à même de comprendre que le procédé selon l'invention ne saurait être limité à des milieux réactionnels particuliers du moment que ces milieux réactionnels comprennent les matériaux indispensables à l'étude des évènements biologiques visés : à titre d'exemple, si les évènements biologiques sont des réactions enzymatiques, le milieu réactionnel devra comprendre les enzymes et les substrats correspondants.

Les procédés de multiplexage selon l'invention permettant de détecter de façon simultanée plusieurs événements biologiques sont particulièrement avantageux dans les applications suivantes :

- **Détection de molécules sécrétées simultanément par des cellules.**

Par exemple, la détection des différentes cytokines sécrétées en réponse à une stimulation de la cellule permet d'affiner la compréhension des mécanismes de communication inter-cellulaires : dans ce cas, les couples de conjugués fluorescents partenaires de FRET seront chacun spécifiques d'une cytokine donnée.

Un autre exemple consiste à détecter et quantifier les peptides béta-amyloïdes : chez les patients atteints par la maladie d'Alzheimer, une augmentation du ratio peptide amyloïde Aβ-42/peptide Aβ-40 a été observée. Les procédés de l'invention permettant de mesurer les différents peptides amyloïdes sécrétés par une même cellule ou culture cellulaire, facilitent grandement la compréhension des mécanismes de développement des plaques amyloïdes et la recherche de médicaments susceptibles d'agir sur la concentration de chacun des peptides amyloïdes. En vue de cette application, les procédés selon l'invention comprendront l'utilisation de conjugués partenaires de FRET permettant de détecter ces peptides.

**• Exploration parallèle de différentes voies de signalisation cellulaire (exemples : dosage simultané de seconds messagers ou autres médiateurs).**

Il est particulièrement intéressant dans l'étude des processus cellulaires de pouvoir obtenir des informations sur les différentes voies de signalisation activées simultanément dans une cellule en réponse à une stimulation extracellulaire.

Les procédés selon l'invention sont particulièrement avantageux pour détecter simultanément les voies de signalisation impliquant les protéines Gs, Gi, Gq ou G11/G12, et les petites protéines G, protéines Ras, Rho, Rab, Ran).

Les voies de signalisation intracellulaires peuvent être mesurées notamment en détectant les évènements suivants :

- stimulation ou inhibition de l'adényl-cyclase par exemple en mesurant la quantité d'AMPc dans la cellule ;
- activation de la phospholipase C, par exemple en mesurant la quantité dans la cellule de composés contenant des inositols (IP3, IP1, IP2, etc.) DAG ;
- activation des kinases couplées aux RCPG (GRK) ;
- translocation de la béta-arrestine.

L'homme du métier est familiarisé avec ces différentes voies de signalisation, qui sont citées qu'à titre limitatif. Des conjugués fluorescents partenaires de FRET spécifiques de chacune de ces voies de signalisation peuvent être mis en oeuvre dans les procédés selon l'invention : ils peuvent être constitués d'un fluorophore et d'anticorps spécifiques d'une molécule caractéristique d'une voie de signalisation donnée (IP3, AMPc etc.) marqués par des partenaires de FRET, ou bien d'une molécule impliquée dans une de ces voies de signalisation, marquée par un composé fluorescent : béta-arrestine couplée à un fluorophore, sous-unité de protéine G, récepteur membranaire couplé à un fluorophore.

Il a été récemment montré que certains récepteurs membranaires, notamment les RCPGs, entraînent l'activation de différentes voies de signalisation intracellulaire en fonction de l'agoniste qui se lie à leur domaine extracellulaire. Ce phénomène est connu sous le terme anglo-saxon de « Agonist Trafficking ».

Les procédés selon l'invention sont idéaux pour étudier ces phénomènes d'« Agonist trafficking » puisqu'ils permettent de mesurer l'activation de l'une ou l'autre des voies de signalisation intracellulaire par un même récepteur membranaire, qui est dans ce cas surexprimé par la cellule par les techniques de biologie moléculaire classique.

**• Etude d'une cascade de signalisation cellulaire.**

La transmission du signal à l'intérieur de la cellule met le plus souvent en oeuvre des cascades d'événements biologiques. Les procédés selon l'invention, en permettant la détection de ces évènements dans une même cellule ou une même population de cellules, fournissent une information bien plus complète que ceux ne mesurant qu'un seul événement : en mettant en évidence l'activation d'au moins deux événements connus pour être impliqués dans une cascade de signalisation, il devient possible de caractériser de manière très précise le mode d'action d'un composé donné. Cela est particulièrement avantageux lorsque l'un des événements biologiques a plusieurs effets dans la cellule.

On peut citer à titre d'exemple non limitatif les cascades de phosphorylation dans la voie de signalisation des MAP Kinases.

**• « Déorphelanisation » de récepteurs dits « orphelins » : associer un récepteur couplé aux protéines G (RCPG) à un ligand et à une voie de signalisation.**

Un certain nombre de récepteurs membranaires ont été identifiés depuis l'avènement du séquençage massif des génomes, notamment du génome humain. Néanmoins pour environ 150 d'entre eux, aucun ligand naturel n'a été identifié et ces récepteurs sont dits « orphelins ». Les procédés selon l'invention facilitent l'identification de ligands de récepteurs orphelins d'une part, et d'autre part permettent de déterminer les voies de signalisation intracellulaires activées par un récepteur orphelin.

Le procédé classique pour « dé-orphelaniser » de tels récepteurs, c'est-à-dire, pour en identifier le ou les ligand(s) naturel(s), consiste à faire surexprimer ledit récepteur par des cellules et à rajouter au milieu de culture un composé dont on veut savoir s'il est un ligand naturel dudit récepteur. La liaison du ligand potentiel peut être facilement mesurée par les techniques connues de l'homme du métier.

Les procédés selon l'invention permettent d'appliquer cette approche à la « dé-orphelanisation » simultanée de plusieurs récepteurs orphelins ; ces procédés permettent en effet de détecter de manière individuelle la liaison d'un ligand à différents récepteurs membranaires exprimés sur une même cellule ou population de cellules : pour cette application particulière des procédés selon l'invention, chacun des couples de conjugués partenaires de FRET permettra de détecter la liaison d'un même ligand potentiel avec les différents récepteurs orphelins. Les molécules impliquées

dans l'événement biologique sont dans ce cas un ligand potentiel d'une part, et différents récepteurs orphelins d'autre part. Les événements biologiques sont constitués par la liaison potentielle du ligand à chacun des récepteurs orphelins. Dans la mesure où le même ligand potentiel est impliqué dans différents évènements biologiques, il est particulièrement avantageux dans cette application précise que les couples de partenaires de FRET aient un élément commun, en l'occurrence le conjugué comprenant ou reconnaissant le ligand.

Une autre approche visant à dé-orphelaniser un récepteur consiste à mesurer l'activation de différentes voies de signalisation intracellulaires par un procédé selon l'invention comme décrit ci-dessus, après la mise en contact de cellules surexprimant un récepteur orphelin avec un ligand potentiel dudit récepteur, ce ligand étant introduit dans le milieu réactionnel au cours d'une étape de stimulation : Ce type d'approche permet d'associer un ligand potentiel, un récepteur orphelin et une voie de signalisation.

**Normalisation de deux événements biologiques l'un par rapport à l'autre, en particulier détection dans le même milieu d'un substrat sous forme modifiée et du substrat total.**

Les procédés selon l'invention permettent également de normaliser l'un par rapport à l'autre deux signaux correspondant à deux événements biologiques donnés ayant lieu dans le même milieu réactionnel. Cela est particulièrement avantageux dans le cas ou l'un des événements biologiques est l'évolution de la quantité d'un substrat modifié -par exemple par une enzyme- et que l'autre événement biologique est l'évolution de la quantité totale de ce substrat (substrat modifié par ladite enzyme + substrat non modifié). Ce type de normalisation du substrat modifié par le substrat total permet de s'affranchir, dans une certaine mesure, de la variabilité des conditions expérimentales d'un milieu réactionnel à l'autre.

A titre d'exemple non limitatif, les procédés selon l'invention sont ainsi applicables pour étudier les modifications enzymatiques suivantes : mono ADP ribosylation ; poly ADP ribosylation ; acétylation ; glutathionylation ; O-glycosylation ; N-glycosylation ; méthylation ; nitration ; phosphorylation ; prénylation ; sumoylation ; ubiquitination ; protéolyse ; biotinylation et glutamylation.

Dans cette mise en oeuvre, un couple de conjugués partenaires de FRET permet de mesurer le substrat modifié et un deuxième couple de conjugués partenaires de FRET permet de mesurer le substrat total. De manière préférée, un des conjugués partenaires de FRET est commun aux deux couples de conjugués partenaires de FRET.

Le schéma de la figure 2 illustre par exemple le principe de détection de l'état de phosphorylation d'une protéine. La méthode repose sur l'utilisation de deux conjugués donneurs et d'un seul conjugué accepteur. Un agent suppresseur de FRET (FRET killer en langue anglaise) reconnaissant spécifiquement une partie d'un des conjugués donneurs est utilisé.

Dans une première étape, la protéine totale est détectée par le FRET entre le donneur $D_1$ et l'accepteur *A,* portés par deux anticorps reconnaissant la protéine (signal $S_T$). Dans une deuxième étape, le signal $S_T$ engendré par la protéine totale est éteint par un agent suppresseur de FRET (Fk) qui reconnaît spécifiquement le donneur $D_1$ et supprime le FRET entre $D_1$ et *A*. Simultanément les sites phosphorylés sont reconnus par un anticorps spécifique qui porte un deuxième donneur $D_2$. Le FRET entre $D_2$ et l'accepteur *A* permet de détecter les protéines phosphorylées (signal $S_P$). Ce format est bien adapté pour le criblage d'inhibiteurs de kinases, par exemple. La détection de la quantité de protéine totale et de la quantité de protéine phosphorylée permet de corriger les variations du nombre de cellules et/ou de la quantité de protéine exprimée dans les différents volumes réactionnels en présence des différents inhibiteurs à tester (opération de normalisation).

L'invention concerne également un nécessaire de composants (kit of parts en langue anglaise) permettant de mettre en oeuvre le procédé selon l'invention : un tel nécessaire comprend au minimum les éléments suivants, fournis ensemble ou séparément :

- deux couples de conjugués fluorescents partenaires de FRET spécifiques de deux événements biologiques ;
- un agent suppresseur du signal de FRET tel que défini à la revendication 1. Un nécessaire de composants selon l'invention peut en outre contenir des instructions décrivant un protocole de mise en contact dans le milieu réactionnel des différents couples de conjugués fluorescents partenaires de FRET, ainsi que des instructions pour la suppression séquentielle des phénomènes de FRET dans le milieu réactionnel par l'ajout de suppresseurs du signal FRET.

**Formats Théoriques** :

Le procédé de multiplexage, selon l'invention, peut être mis en oeuvre selon plusieurs formats :

*(i)* Format 1 : reconnaissance spécifique d'une partie du conjugué donneur par un agent suppresseur du signal FRET et suppression du FRET ;

*(ii)* Format 2 : reconnaissance spécifique d'une partie du conjugué accepteur par un agent suppresseur du signal

FRET et suppression du FRET ;

*(iii)* Format 3 : reconnaissance spécifique d'une partie du conjugué accepteur par un agent suppresseur du signal FRET et suppression du signal de FRET.

***(i) Format 1 : reconnaissance spécifique d'une partie du conjugué donneur par un agent suppresseur du signal FRET et suppression du FRET.***

Le schéma de la figure 3 illustre un exemple de mise en oeuvre de ce format de multiplexage pour la détection de deux analytes *X* et *Y.* Ce format repose sur l'utilisation de :

- deux donneurs, $D_1$ et $D_2$, excitables par un même stimuli (par exemple une même longueur d'onde d'excitation lumineuse),
- un accepteur *A* commun aux deux donneurs,
- et un agent suppresseur du signal FRET capable de reconnaître spécifiquement un des deux donneurs et de supprimer le FRET entre ce donneur et l'accepteur.

Dans une première étape, l'analyte *X* est détecté par le FRET entre le donneur $D_1$ et l'accepteur *A* portés par deux anticorps spécifiques de l'analyte *X.* Dans une deuxième étape, le produit *Fk* reconnaît spécifiquement le donneur $D_1$ et supprime le FRET entre $D_1$ et l'accepteur *A.* La suppression du FRET éteint le signal engendré par la présence de l'analyte *X*. Simultanément, l'analyte *Y* est détecté par le FRET entre le donneur $D_2$ et l'accepteur *A,* portés par deux anticorps spécifiques de l'analyte *Y.*
Ce format est mis en oeuvre dans l'exemple 2.

***(ii) Format 2 : reconnaissance spécifique d'une partie du conjugué accepteur par un agent suppresseur du signal FRET et suppression du FRET.***

Le schéma de la figure 4 illustre un exemple de mise en oeuvre de ce format de multiplexage pour la détection de deux analytes *X* et *Y.* Ce format repose sur l'utilisation de :

- deux accepteurs, $A_1$ et $A_2$, détectables à la même longueur d'onde ;
- un donneur *D* commun aux deux accepteurs ;
- et un agent suppresseur du signal FRET capable de reconnaître spécifiquement un des deux accepteurs et de supprimer le FRET entre le donneur et cet accepteur.

Dans une première étape, l'analyte *X* est détecté par le FRET entre le donneur *D* et l'accepteur $A_1$ portés par deux anticorps spécifiques de l'analyte *X.* Dans une deuxième étape, le produit *Fk* reconnaît spécifiquement l'accepteur $A_1$ et supprime le FRET entre *D* et $A_1$. La suppression du FRET éteint le signal engendré par la présence de l'analyte *X*. Simultanément, l'analyte *Y* est détecté par le FRET entre le donneur *D* et l'accepteur $A_2$, portés par deux anticorps spécifiques de l'analyte *Y.*
Ce format est illustré par l'exemple 5.

***(iii) Format 3 : reconnaissance spécifique d'une partie du conjugué accepteur par un agent suppresseur du signal FRET et suppression du signal de FRET.***

Le schéma de la figure 5 illustre un exemple de mise en oeuvre de ce format de multiplexage pour la détection de deux analytes *X* et *Y.* Ce format repose sur l'utilisation de :

- deux accepteurs, $A_1$ et $A_2$, détectables à la même longueur d'onde ;
- un donneur *D* commun aux deux accepteurs ;
- et un agent suppresseur du signal FRET capable de reconnaître spécifiquement un des deux accepteurs et de supprimer le signal de FRET de cet accepteur.

Dans une première étape, l'analyte *X* est détecté par le FRET entre le donneur D et l'accepteur $A_1$ portés par deux anticorps spécifiques de l'analyte *X.* Dans une deuxième étape, le produit *Fk* reconnaît spécifiquement l'accepteur $A_1$ et supprime le signal de FRET de $A_1$. Simultanément, l'analyte *Y* est détecté par le FRET entre le donneur *D* et l'accepteur $A_2$, portés par deux anticorps spécifiques de l'analyte *Y.*

L'invention va être décrite plus en détail par les exemples illustratifs ci-après dans lesquels il est fait référence aux figures 6A, 6B, 7-14 dont une brève description est donnée ci-après, les figures 1 à 5 représentant les schémas

mentionnés précédemment.

**Brève description des figures** :

Figure 1 : schéma illustrant le phénomène de compétition.

Figure 2 : schéma illustrant le principe de détection de l'état de phosphorylation d'une protéine.

Figure 3 : schéma illustrant un exemple de mise en oeuvre du format 1 de multiplexage pour la détection de deux analytes *X* et *Y*.

Figure 4 : schéma illustrant un exemple de mise en oeuvre du format 2 de multiplexage pour la détection de deux analytes *X* et *Y*.

Figure 5 : schéma illustrant un exemple de mise en oeuvre du format 3 de multiplexage pour la détection de deux analytes *X* et *Y*.

Figure 6A :_Suppression du signal de FRET par un anticorps anti-cryptate : Après une étape de détection de la protéine PS2-GST par le FRET entre le TBP et l'accepteur A647 (graphe I), des concentrations croissantes d'anti-TBP ont été ajoutées au volume réactionnel et le signal de l'accepteur engagé dans le FRET a été suivi au cours du temps (graphe II. Losanges : antiTBP 6nM ; triangles : antiTBP 30nM ; carrés : antiTBP 150nM). A des concentrations adaptées, le signal de l'accepteur engagé dans le FRET disparaît rapidement (<30min). Cette extinction du signal de l'accepteur découle de l'interaction entre le TBP et l'antiTBP qui supprime le FRET.

Figure 6B : La même expérience que celle présentée sur la figure 6A a été conduite en utilisant le donneur TBP5COOH et le même accepteur A647. Après avoir détecté PS2-GST par le FRET entre le TBP5COOH et l'accepteur A647 (graphe I), des concentrations croissantes d'antiTBP ont été ajoutées au volume réactionnel et le signal de l'accepteur engagé dans le FRET a été suivi au cours du temps (graphe II : cercles : absence d'antiTBP ; losanges : antiTBP 6nM ; triangles : antiTBP 30nM ; carrés : antiTBP 150nM). Le signal de l'accepteur engagé dans le FRET ne diminue pas, quelle que soit la concentration en antiTBP.

Figure 7 : Exemple de multidétection de deux protéines par reconnaissance d'un donneur et suppression du FRET : Après une étape de détection de la protéine PS2-GST par le FRET entre le TBP et l'accepteur XL665 (graphe I), l'antiTBP et le deuxième anticorps pour la détection du TNFα sont ajoutés. L'antiTBP reconnaît spécifiquement le TBP, supprime le FRET entre le TBP et la XL665 et éteint ainsi le signal de la PS2-GST. Simultanément, le TNFα est détecté par le FRET entre le TBP5COOH et la XL665 (graphe II, barres foncées), portés par deux anticorps spécifique du TNFα. Les signaux obtenus dans l'essai en multidétection (graphe II, barres foncées) sont comparables à ceux obtenus dans un essai de simple détection (graphe II, barres gris clair).

Figure 8 : Exemple de multidétection des peptides amyloides Aβ-40 et Aβ-42 par reconnaissance d'un donneur et suppression du FRET : Après une étape de détection du peptide Aβ-42 par le FRET entre le TBP et l'accepteur d2 (graphe I), l'antiTBP et l'anticorps pour la détection spécifique du Aβ-40 sont ajoutés. L'antiTBP reconnaît spécifiquement le TBP, supprime le FRET entre le TBP et d2 et éteint ainsi le signal du Aβ-42. Simultanément, le Aβ-40 est détecté par le FRET entre un autre type de cryptate d'europium K (porté par un anticorps spécifique du Aβ-40) et d2 (graphe II, barres foncées). Les signaux obtenus dans l'essai en multidétection (graphe II, barres foncées) sont comparables à ceux obtenus dans un essai de simple détection (graphe II, barres gris clair).

Figure 9 : Après une étape de détection du TNFα par le FRET entre un donneur cryptate d'europium et la sonde accepteur aS1-Cy5 (graphe I), des concentrations croissantes d'oligonucléotide eS1 ont été ajoutées au volume réactionnel et le signal de l'accepteur engagé dans le FRET a été suivi au cours du temps (graphe II : cercles : eS1 8nM ; losanges : eS1 20nM ; triangles : eS1 100nM ; carrés : eS1 200nM). Les oligonulcéotides aS1 et eS1 sont complémentaires. Les concentrations finales de eS1 et de aS1-Cy5 sont de 2nM et 4nM respectivement. A des concentrations adaptées, le signal de l'accepteur engagé dans le FRET disparaît complètement et rapidement (<30min).

Figure 10 : Exemple de multidétection de deux protéines par reconnaissance d'une partie du conjugué accepteur et suppression du FRET. Après une étape de détection de TNFα par mesure du FRET (graphe I), les produits eS1 et aS2-Cy5 sont ajoutés. L'oligonucléotide eS1 reconnaît spécifiquement la séquence aS1 et découple la sonde aS1-Cy5 de l'anticorps anti TNFα, supprimant ainsi le FRET dû à la présence de TNFα. Simultanément, la sonde aS2-Cy5 s'hybride à la séquence S2, ce qui permet la détection de la protéine PS2-GST. Les signaux obtenus dans l'essai en multidétection (graphe II, barres foncées), sont similaires à ceux obtenus dans un essai en simple détection (graphe II, barres claires).

Figure 11 : Utilisation d'un ion divalent ou d'un agent chélatant comme agent suppresseur de FRET

Figure 12 : Utilisation de l'acide urique comme agent suppresseur de FRET

Figure 13 : Evolution du signal de FRET représentatif de la quantité d'IP1 produit par la cellule en fonction de la concentration d'acétylcholine présente dans le milieu. La diminution du signal de FRET résulte de la production par

la cellule d'IP1, par un phénomène de compétition (voir exemple 8)

Figure 14 : Evolution du signal de FRET représentatif de la quantité de cAMP produite par la cellule en fonction de la concentration d'acétylcholine présente dans le milieu..La diminution du signal de FRET résulte de la production par la cellule de cAMP, par un phénomène de compétition (voir exemple 8)

Les abréviations utilisées dans ces exemples ont les significations ci-après :

- PS2-GST : désigne une protéine de fusion comprenant la protéine PS2 (décrite notamment dans le brevet EP 345 315) et la protéine GST (glutathion-S-transférase). Une telle protéine de fusion est produite selon les techniques classiques de biologie moléculaire, par exemple à l'aide d'un système d'expression tel qu'un plasmide comprenant la séquence d'acides nucléiques codant pour cette protéine de fusion. Les séquences de la PS2 et de la GST sont connues.
- TBP : désigne le cryptate tris-bipyridine.
- TBP5COOH : désigne cryptate tris-bipyridine penta-acide.
- PBP4COOH : désigne le cryptate pyridine-bispyridine tétra-acide.
- A647 : désigne le fluorophore alexa647 de la société Molecular Probe.
- XL665 : Allophycocyanine réticulée, utilisée comme fluorophore accepteur, commercialisée par CIS bio international.
- d2 : composé fluorescent accepteur ayant les mêmes caractéristiques photophysiques que XL665.
- Cy5 : Cyanine 5, fluorophore accepteur commercialisé par la société GE Healthcare.

Dans ces exemples, on a utilisé les anticorps suivants qui sont spécifiques de divers antigènes connus et ont été préparés à l'aide des techniques classiques :

- Anticorps GSS11 : désigne un anticorps monoclonal spécifique de la protéine GST.
- Anticorps BC04 : désigne un anticorps monoclonal spécifique de la protéine PS2.
- Anticorps IPM3 : désigne un anticorps monoclonal spécifique du TNFα, différent de l'anticorps IPM2.
- Anticorps IPM2 : désigne un anticorps monoclonal spécifique du TNFα, différent de l'anticorps IPM3.
- Anticorps G211 : désigne un anticorps monoclonal spécifique du peptide amyloïde Aβ 1-42
- Anticorps 13E9 : désigne un anticorps monoclonal spécifique du peptide amyloïde Aβ 1-40
- Anticorps 1E8 : désigne un anticorps monoclonal reconnaissant à la fois les peptides amyloïdes Aβ 1-42 et 1-40.

**<u>Exemple 1 : Suppression du FRET entre un donneur et un accepteur par un agent suppresseur du signal FRET reconnaissant spécifiquement une partie du conjugué donneur (figures 6A et 6B)</u>**

Un anticorps qui reconnaît spécifiquement un complexe de terre rare, le cryptate d'europium tris-bipyridine TBP a été produit selon les techniques classiques (anticorps antiTBP). Dans cet exemple, il est démontré que l'antiTBP reconnaît spécifiquement le cryptate d'europium TBP et supprime le FRET entre le TBP et son accepteur.

***Protocole***

Deux expériences identiques visant à détecter une protéine de fusion *PS2-GST* ont été conduites. Dans chacune des expériences, une structure particulière de cryptate d'europium a été utilisée comme donneur de FRET : soit le cryptate tris-bipyridine *TBP* soit le cryptate tris-bipyridine penta-acide *TBP5COOH* ayant les formules ci-après :

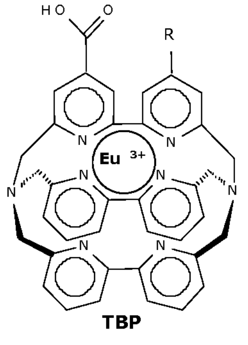

**TBP**

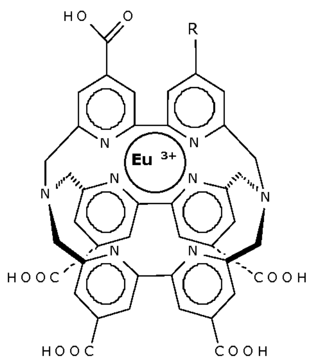

**TBP5COOH**

R est un groupe réactionnel classique permettant le couplage du cryptate avec la substance à marquer.
Ces deux expériences sont illustrées par les figures 6A et 6B.

La protéine de fusion *PS2-GST* est distribuée dans une plaque 96 puits. Sa présence est détectée par un FRET entre le donneur (le *TBP* ou le *TBP5COOH*) (porté par l'anticorps *GSS11*) et l'accepteur *A647* (porté par l'anticorps *BC04),* après incubation pendant 16h à température ambiante. Le signal émis par l'accepteur engagé dans le FRET (c'est-à-dire le signal de l'accepteur suite à l'excitation sélective du donneur) est appelé S (665nm).

Des concentrations croissantes d' *antiTBP* sont ensuite ajoutées au temps *t=0* et le signal S *(665nm)* de l'accepteur engagé dans le FRET a été suivi au cours du temps (température ambiante, ≈ 20°C).

*Concentrations finales :*

***PS2-GST*** 500pM; ***GSS11*** 1,5nM; ***BC04*** 0,677nM;
***antiTBP*** 0,6nM, 30nM et 150nM

*Volume réactionnel final :* 200μl
*Tampon :* phosphate 100mM pH 7,0, KF 400mM, BSA 0,1%

Les signaux ont été détectés sur un Rubystar (BMG Labtechnologies, excitation laser 337nm).

***Résultats***

La figure 6A illustre les résultats de l'expérience conduite avec le *TBP.* La protéine à détecter (*PS2-GST*) est reconnue par deux anticorps spécifiques portant le donneur *TBP* et l'accepteur *A647.* Suite à la reconnaissance de la protéine par les deux anticorps, le *TBP* et son accepteur se retrouvent en proximité. En excitant sélectivement le *TBP* (337nm), son énergie d'excitation est alors en partie transférée à l'accepteur par FRET. Ce transfert d'énergie provoque une émission de fluorescence de l'accepteur (le signal *S* (*665nm*)). L'apparition de l'émission de l'accepteur suite à l'excitation sélective du donneur permet de détecter la protéine.

Après avoir ainsi détecté la protéine (figure 6A, graphe I), l'anticorps *antiTBP* est ajouté, au temps *t=0,* au volume réactionnel. On observe alors l'extinction du signal *S(665nm),* c'est-à-dire du signal de l'accepteur engagé dans le FRET (figure 6A, graphe II).

L'extinction de ce signal n'est pas due à une interaction de l'*antiTBP* avec l'accepteur utilisé, mais découle uniquement de la suppression du FRET suite à l'interaction spécifique de l'*antiTBP* avec le *TBP.* Pour prouver cela, la même expérience a été conduite en utilisant un donneur différent (le *TBP5COOH* au lieu du *TBP*), et le même accepteur *A647* (figure 6B). Le FRET entre le *TBP5COOH* et le *A647* permet de détecter la protéine (figure 6B, graphe I). La présence de l'*antiTBP* ne modifie pas ce FRET : le signal *S (665nm)* ne diminue pas, quelle que soit la concentration en *anti-TBP* (figure 6B, graphe II). La disparition du signal de l'accepteur observée avec le *TBP* (figure 6A, graphe II) est donc bien due à la suppression du FRET entre le donneur *TBP* et l'accepteur suite à la reconnaissance spécifique du *TBP* par l'*antiTBP.*

**Exemple 2 : Détection multiplexée de deux protéines, PS2-GST et TNFα - Suppression du FRET par un agent se liant à un des fluorophores donneurs (figure 7).**

Cet exemple repose sur l'utilisation de deux donneurs excitables à la même longueur d'onde (le *TPB* et le *TBP5COOH*) et un seul accepteur commun détectable à 665nm (la protéine fluorescente *XL665*). L'agent suppresseur du signal FRET est l'anticorps *antiTBP,* qui reconnaît spécifiquement le *TBP* et supprime le FRET entre le *TBP* et son accepteur comme cela est montré dans l'exemple précédent.

***Protocole***

L'expérience est illustré par la figure 7, pour la détection de deux analytes : la protéine *PS2-GST* et la protéine *TNFα.* Une gamme de concentrations de *TNFα* a été mesurée, après une première étape de détection de la protéine *PS2-GST* et extinction du signal de FRET correspondant.

*Expérience de multiplexage*

Une gamme de concentrations de *TNFα* en présence d'une concentration fixe de *PS2-GST* est distribuée dans une plaque 96 puits.

La protéine *PS2-GST* est tout d'abord détectée par le FRET entre le cryptate d'europium *TBP* (porté par l'anticorps *GSS11*) et l'accepteur *XL665* (porté par l'anticorps *BC04),* après incubation *pendant 16h.* à température ambiante.

Les composés suivants sont ensuite ajoutés aux volumes réactionnels :

- l'*antiTBP* pour supprimer le FRET entre le *TBP* et la *XL665* et donc le signal dû à la présence de la protéine *PS2-GST;*
- et le deuxième anticorps pour la détection de la protéine *TNF*$\alpha$ : l'anticorps *IPM3* portant l'accepteur *XL665* (l'anticorps *IPM2* portant le donneur cryptate d'europium *TBP5COOH* étant distribué dans l'étape I).

Après une incubation de 4h à température ambiante (pour permettre la reconnaissance du *TNF*$\alpha$ par l'anticorps spécifique *IPM3*), les signaux obtenus dans les puits contenant les différentes concentrations de *TNF*$\alpha$ sont mesurés.

*Expérience de contrôle : détection simple*

Les signaux ainsi obtenus dans l'essai de multiplexage sont comparés avec les signaux obtenus dans un essai de détection simple du *TNF*$\alpha$ (c'est-à-dire la même expérience illustrée par la figure 7, mais en l'absence de *PS2-GST*).

*Concentrations finales des protéines et des anticorps:*

***PS2-GST*** 500pM ; ***GSS11*** 1,5nM ; ***BC04*** 0,667nM ;
***antiTBP*** 150nM ;
**TN*F***$\alpha$ 0*,* 10, 20, 40, 80 et 120pM ; ***IPM2*** 0,33nM ; ***IPM3*** 0,8nM

*Volume réactionnel final :* 200$\mu$l
*Tampon :* phosphate 100mM pH 7,0, KF 400mM, BSA 0,1%

Les signaux ont été détectés sur un Rubystar (excitation laser 337nm).

***Résultats***

Dans une première étape, la protéine *PS2-GST* a été détectée par le FRET entre le *TBP* et l'accepteur *XL665.* Le signal du FRET *S(665nm),* engendré par la présence de la protéine *PS2-GST,* est représenté par la barre de l'histogramme I, figure 7. Dans une deuxième étape, ont été ajoutés aux volumes réactionnels l'anticorps *antiTBP* pour supprimer le FRET dû à la protéine *PS2-GST,* et le deuxième anticorps pour la détection du *TNF*$\alpha$*.* Les signaux détectés après l'addition de ces composés sont représentés par les barres foncées de l'histogramme II, figure 7.

Une expérience de contrôle a permis de valider cette expérience de multiplexage : Les résultats obtenus en multiplexage sont comparés avec ceux obtenus dans un essai de détection simple. Plus précisément la même expérience que celle illustrée dans le schéma de la figure 7 a été conduite, mais en absence de la protéine *PS2-GST.* Dans cet essai à détection simple, aucun signal n'est à éteindre et les différentes concentrations de *TNF*$\alpha$ sont détectées directement. Les signaux obtenus sont représentés par les barres claires de l'histogramme II, figure 7 Les signaux issus du multiplexage sont comparables à ceux issus de la détection simple.

**<u>Exemple 3 : Détection multiplexée des peptides amyloïdes Aβ-40 et Aβ-42 - Suppression du FRET par un agent reconnaissant un des fluorophores donneurs (figure 8)</u>**

Les peptides amyloïdes *A*$\beta$*-40* et *A*$\beta$*-42* sont impliqués dans la maladie d'Alzheimer. Dans cette maladie, la production du peptide neurotoxique *A*$\beta$*-42* est favorisée par rapport à celle du *A*$\beta$*-40.* Le peptide *A*$\beta$*-42* se différentie du peptide *A*$\beta$*-40* par la présence de deux aminoacides supplémentaires à une extrémité. La figure 8 illustre la détection multiplexée des peptides amyloïdes *A*$\beta$*-40* et A$\beta$*-42*. L'expérience repose sur l'utilisation :

- de deux conjugués donneurs (un conjugué pour la reconnaissance spécifique du peptide *A*$\beta$*-42* et portant un cryptate d'europium *TBP* et un conjugué pour la reconnaissance spécifique du peptide *A*$\beta$*-40* et portant un cryptate d'europium différent, le *TBP5COOH,* également dénommé ci-après cryptate d'europium K ;
- d'un seul conjugué accepteur pouvant reconnaître les deux peptides *A*$\beta$*-42* et *A*$\beta$*-42* ;
- et de l'agent suppresseur du signal FRET *antiTBP.*

***Protocole***

L'expérience est illustrée par la figure 8 : une gamme de peptide *A*$\beta$*-40* a été mesurée, après une étape de détection du peptide *A*$\beta$*-42* et extinction du signal de FRET correspondant.

*Expérience de multiplexage*

Une gamme de concentrations de *Aβ-40* en présence d'une concentration fixe de *Aβ-42 est* distribuée dans une plaque 96 puits. Les deux peptides sont reconnus par un anticorps (le *1E8*) portant un accepteur (l'accepteur d2) commun aux deux donneurs utilisés dans cette expérience. Le peptide *Aβ-42* est également reconnu par un anticorps spécifique (le *G211*) portant un cryptate d'europium *TBP.* Après avoir ainsi détecté le peptide *Aβ-42* par le FRET entre le *TBP* et l'accepteur d2, les composés suivants sont ajoutés aux volumes réactionnels :

- l'*antiTBP* pour supprimer le FRET entre le *TBP* et l'accepteur d2 et donc le signal dû à la présence du *Aβ-42 ;*
- et un anticorps (le *13E9*) pour la détection spécifique du *Aβ-40* ; cet anticorps portant le cryptate d'europium *K* qui n'est pas reconnu par l'*antiTBP.*

Après une incubation de 3h à 4°C (pour permettre la reconnaissance du *Aβ-40* par son anticorps), les signaux obtenus sont mesurés dans les puits contenant les différentes concentrations de *Aβ-40.*

*Expérience de contrôle : détection simple*

Les signaux ainsi obtenus dans l'essai de multiplexage sont comparés avec les signaux obtenus dans un essai de détection simple du *Aβ-40* (c'est à dire la même expérience que celle illustrée par la figure 8, mais en absence du peptide *Aβ-42*).

*Concentrations finales des peptides et des anticorps* :

***Aβ-42*** 3200pg/ml ;
***Aβ-40*** 200, 400, 800 et 1200pg/ml ;
***1E8*** 3,3nM ; ***G211*** 0,8nM ; ***13E9*** 0,34nM

*Volume réactionnel final :* 100μl
*Tampon :* phosphate 50mM pH 7,4, KF 250mM, BSA 0,2%, Tween 0,05%, EDTA 0,25mM

Les signaux ont été détectés sur un Rubystar (excitation laser 337nm).

***Résultats***

Dans une première étape, le peptide *Aβ-42* est détecté par le FRET entre le *TBP* et l'accepteur *d2.* L'accepteur *d2* est porté par un anticorps reconnaissant les deux peptides, tandis que le donneur *TBP* est porté par un anticorps reconnaissant spécifiquement le peptide *Aβ-42.* Le signal *S (665nm)* de l'accepteur impliqué dans le FRET est représenté par la barre de l'histogramme I, figure 8.

Dans une deuxième étape, ont été ajoutés aux volumes réactionnels l'*antiTBP* (pour supprimer le signal dû au peptide *Aβ-42*) et un anticorps qui reconnaît spécifiquement le peptide *Aβ-40* et qui porte le cryptate d'europium *K* différent du *TBP.* Les signaux détectés après l'addition de ces composés sont représentés par les barres foncées de l'histogramme II, figure 8.

Pour valider les signaux obtenus dans cette expérience de multiplexage, la même expérience que celle illustrée figure 8 a été conduite, mais en l'absence du peptide *Aβ-42.* Dans cet essai de détection simple, aucun signal n'est à éteindre et les différentes concentrations de *Aβ-40* sont détectées directement. Les signaux obtenus sont représentés par les barres claires de l'histogramme II, figure 8. Les signaux issus du multiplexage sont comparables à ceux issus de la détection simple.

**Exemple 4** : **Suppression du FRET entre un donneur et un accepteur par un agent suppresseur du signal FRET agissant par découplage du fluorophore accepteur (figure 9).**

Cet exemple est relatif à un agent suppresseur du signal FRET qui agit en formant un complexe non-covalent avec l'un des conjugués partenaire de FRET, et va le découpler de l'événement biologique dont il est spécifique, par un mécanisme de compétition.

***Nature et mode d'action de cet agent suppresseur du signal FRET***

Cet agent suppresseur du signal FRET est un oligonucléotide. La figure 9 illustre son mode d'action.

Dans cet exemple, le conjugué fluorescent accepteur est constitué d'une petite molécule organique fluorescente (une cyanine Cy5) couplée à un oligonucléotide de séquence *aS1.* Cette séquence s'hybride à une séquence complémentaire *S1* couplée à un anticorps spécifique de la protéine à détecter.

Après avoir détecté l'événement biologique par le FRET entre un conjugué donneur et ce conjugué accepteur, un oligonucléotide *eS1* de séquence complémentaire à *aS1* est ajouté au volume réactionnel. La séquence *eS1* reconnaît spécifiquement la séquence *aS1* et déplace ainsi le conjugué fluorescent accepteur (*aS1-Cy5*) de l'anticorps spécifique de la protéine à détecter. Le déplacement du conjugué fluorescent accepteur supprime le FRET entre le conjugué donneur et le conjugué accepteur.

L'oligonucléotide *eS1* constitue un exemple d'agent suppresseur du signal FRET qui reconnaît spécifiquement une partie du conjugué accepteur et supprime le FRET. La spécificité de reconnaissance repose dans ce cas sur l'appariement de deux séquences complémentaires d'ADN.

***Protocole***

La protéine *TNFα* a été distribuée dans une plaque 96 puits. Sa présence a été détectée par un FRET entre un donneur cryptate d'europium (porté par l'anticorps *IPM2*) et le conjugué accepteur *aS1-Cy5* (qui se lie par appariement des brins complémentaires à l'anticorps *IPM3,* comme précisé ci-dessus). *S(665nm)* est le signal de l'accepteur engagé dans le FRET.

Après avoir détecté la protéine, des concentrations croissantes d'oligonucléotide *eS1* ont été apportées au temps *t=0* et le signal *S(665nm)* de l'accepteur engagé dans le FRET a été suivi au cours du temps (température ambiante).

*Concentrations finales protéine et anticorps:*

***TNFα*** 150pM ; ***IPM2*** 0,33nM ; ***IPM3*** 1,07nM.

*Concentrations finales oligonucléotides :*

***S1*** 2nM; ***aS1-Cy5*** 4nM ; ***eS1*** 8, 20, 100 et 200nM

*Séquences :*

**S1** 3'TTGATTTCGACAATTGTT5'
**aS1** 5'AACTAACGCTGGTAACAAGTCGTAC3'
**eS1** 3'TTgATtGCgACCATtGTTCAgCATG5'
les nucléotides en caractère minuscule sont des LNA (Locked Nucleic Acids)

*Volume réactionnel final :* 200μl
*Tampon :* HEPES 50mM pH 7,0, KF 400mM, BSA 0,1% ; température ambiante

Les signaux ont été détectés sur un Rubystar (excitation laser 337nm).

***Résultats***

L'hybridation entre le conjugué accepteur *aS1-Cy5* et la séquence *S1* portée par l'anticorps, permet de détecter la protéine par FRET (figure 9, graphe I). Après avoir ainsi détectée la protéine, le produit *eS1* est ajouté au volume réactionnel. En présence du produit *eS1,* on observe alors l'extinction du signal *S(665nm)* du conjugué accepteur engagé dans le FRET (figure 9, graphe II). L'extinction de ce signal est due au déplacement de du conjugué *aS1-Cy5* par *eS1.* Le déplacement du conjugué accepteur supprime le FRET entre les conjugués donneur et accepteur.

**Exemple 5 : Détection multiplexée de deux protéines, TNFα et PS2-GST - Suppression du FRET par découplage d'un des fluorophores accepteurs (figure 10)**

Cet exemple repose sur l'utilisation d'un seul donneur D (un cryptate d'europium) et de deux conjugués accepteurs (*aS1-Cy5* et *aS2-Cy5*). Les conjugués accepteurs sont constitués d'une cyanine couplée à une séquence oligonucléotidique aS1 d'une part et aS2 d'autre part. L'agent suppresseur du signal FRET est un oligonucléotide qui reconnaît spécifiquement une partie d'un des conjugués accepteurs et supprime le FRET, selon le mode d'action illustré dans l'exemple précédent.

***Protocole***

La figure 10 illustre le principe de l'expérience de multidétection, pour la détection de deux protéines : *TNFα*

et *PS2-GST.* Une gamme de concentrations de *PS2-GST* a été mesurée après une étape de détection de la protéine *TNFα* et extinction du signal de FRET correspondant.

*Expérience de multiplexage*

Une gamme de concentrations *PS2-GST* est distribuée dans une plaque 96 puits en présence d'une concentration fixe de *TNFα.*

Pour la détection de la première protéine (*TNFα*), les composés suivants sont introduits dans chaque puits :

- un anticorps (*IPM2*) portant un donneur cryptate d'europium
- et un conjugué constitué d'un anticorps (*IPM3*) portant un oligonucléotide *S1.* Le conjugué accepteur est constitué d'une *Cy5* couplée à un oligonucléotide *aS1* qui s'hybride spécifiquement avec *S1.*

De façon similaire, pour la détection de la deuxième protéine (*PS2-GST*) nous avons utilisé

- un anticorps (*GSS11*) portant un donneur cryptate d'europium
- et un conjugué accepteur constitué d'un anticorps *(BC04)* portant un oligonucléotide S2. Le conjugué accepteur est constitué d'une *Cy5* couplée à un oligonucléotide aS2 qui s'hybride spécifiquement avec S2.

Dans une première étape, tous les anticorps ainsi que le conjugué accepteur *aS1-Cy5* ont été distribués dans les puits. Après incubation *pendant 16h.* à température ambiante, la protéine *TNFα* est détectée en mesurant le signal de FRET émis par chaque puits.

Dans une deuxième étape, les composés suivants sont introduits dans les puits :

- l'oligonucléotide *eS1,* de séquence parfaitement complémentaire à *aS1*
- et le conjugué accepteur *aS2-Cy5.*

L'oligonucléotide *eS1* s'hybride spécifiquement avec *aS1* et déplace ainsi la sonde *aS1-Cy5* du conjugué accepteur ; le FRET n'a plus lieu et donc le signal de la protéine *TNFα* disparaît. Simultanément, la sonde *aS2-Cy5* s'hybride avec S2 et permet d'« allumer » le conjugué accepteur, c'est-à-dire de détecter la protéine *PS2-GST.*

Une fois ajouté l'agent suppresseur du signal FRET *eS1* et la sonde *aS2-Cy5,* les signaux de FRET sont mesurés après un temps d'incubation de 30 minutes à température ambiante.

*Expérience de contrôle : détection simple*

Les signaux ainsi obtenus dans l'essai de multiplexage sont comparés avec les signaux obtenus dans un essai de détection simple de la *PS2-GST* (c'est-à-dire la même expérience que celle illustrée dans la figure 10, mais en absence de *TNFα*).

*Concentrations finales protéine et anticorps:*

***TNFα* 80pM** ; ***IPM2*** 0,33nM ; ***IPM3*** 1,07nM ;
***PS2-GST*** 0, 75, 150, 250, 350 et 500pM ; ***GSS11*** 1nM ; *BC04* 0,69nM *Concentrations finales oligonucléotides :*
***S1*** 2nM; ***aS1-Cy5*** 4nM ; *eS1* 100nM
**S2** 2nM ; ***aS2-Cy5*** 20nM

*Séquences :*

***S1*** 3'TTGATTTCGACAATTGTT5'
***aS1*** 5'AACTAACGCTGGTAACAAGTCGTAC3'
***eS1*** 3'TTgATtGCgACCATtGTTCAgCATG5'
les nucléotides en caractère minuscule sont des LNA (Locked Nucleic Acids) ;
***S2*** 3'ATATTGGTAGTTCCAGAT5'
***aS2*** 5'TATAACCATCAAGGTCTA3'

*Volume réactionnel final :* 200μl.
*Tampon :* HEPES 50mM pH 7.0, KF 400mM, BSA 0.1% ; température ambiante.

Les signaux ont été détectés sur un RUBYstar (excitation laser 337nm).

***Résultats***

Dans une première étape, la protéine *TNF$\alpha$* est détectée via l'hybridation de la sonde *aS1-Cy5* avec *S1*: le signal *S(665nm)* du conjugué accepteur *aS1-Cy5* engagé dans le FRET est représenté dans l'histogramme I, figure 10. Dans une deuxième étape est ajouté l'oligonucléotide suppresseur du signal FRET *eS1* et le conjugué accepteur *aS2-Cy5*. Les signaux détectés après l'addition de ces produits sont représentés par les barres foncées de l'histogramme II, figure 10.

Pour valider cette expérience de multiplexage, les signaux ainsi obtenus sont comparés avec ceux obtenus dans un essai à détection simple, c'est-à-dire un essai comme celui illustré figure 10, mais en absence de la protéine *TNF$\alpha$*. Dans cet essai de détection simple, aucun signal n'est à éteindre et les différentes concentrations de *PS2-GST* sont directement détectées. Les signaux de l'essai de détection simple sont représentés par les barres claires de l'histogramme II, figure 10: ils sont comparables avec ceux obtenus dans l'essai de multidétection.

**Exemple 6 : Utilisation d'un ion divalent ou d'un agent chélatant comme agent suppresseur du FRET entre un donneur et un accepteur (figure 11)**

Dans cet exemple, il est démontré que l'ion manganèse ou un agent chélatant de type EDTA sont capables de supprimer sélectivement le FRET existant entre un donneur de type complexe de terre rare et un accepteur.

***Protocole***

Deux expériences identiques visant à détecter une protéine *GST-biotine* ont été conduites. Dans chacune des expériences, un complexe de terre rare particulier a été utilisé comme donneur de FRET : soit le cryptate tris-bipyridine *TBP* soit un chélate de terbium commercialisé sous forme conjugué à un anticorps anti-GST par la société Invitrogen (Cat n°PV3568).

La protéine GST biotinylée a été distribuée dans une plaque 96 puits. Sa présence a été détectée par un FRET entre le donneur (le TBP ou le chélate de terbium) (portés par un anticorps anti-GST) et l'accepteur d2 (porté par la streptavidine), après incubation pendant 16h à température ambiante en présence ou en absence de 20mM de chlorure de manganèse ($MnCl_2$) ou d'EDTA. On effectue une mesure de fluorescence en temps résolu (td = 50$\mu$s, tg = 400$\mu$s) à 620nm et 665nm (respectivement E620 et E665) sur un appareil Rubystar® (BMG Labtech). A partir des valeurs d'intensité de fluorescence, E620 et E665, on calcule le rapport d'intensité E665/E620 dénommé ratio que l'on multiplie par 10 000 pour plus de commodité. Les valeurs de delta F sont calculées par rapport au ratio mesuré dans un puits négatif ne contenant pas la GST biotinylée (cf G. Mathis, Clin. Chem. 39 (1993) 1953 et la demande WO92/13264). Afin de comparer les deltas F obtenus dans les différents milieux pour un même donneur, ceux-ci sont normalisés par rapport au delta F obtenu en tampon (100%).

*Concentrations finales :*

***GST biotinylée*** 1,25nM; ***anti-GST*** 2nM; **Streptavidine** 5nM;
***$MnCl_2$ ou EDTA*** 20mM

*Volume réactionnel final :* 200$\mu$l
*Tampon :* phosphate 100mM pH 7,0, KF 400mM, BSA 0,1%

***Résultats***

La figure 11 illustre les résultats obtenus dans les deux expériences. L'ajout de 20mM de $MnCl_2$ ou d'EDTA ne modifie pas le signal de FRET (delta F normalisé) obtenu avec le donneur TBP en présence de GST biotinylée. A l'inverse, le $MnCl_2$ ou l'EDTA agissent comme suppresseurs de FRET (signal de FRET inhibé de plus de 70%) si l'on utilise le chélate de terbium comme donneur pour détecter la GST biotinylée. Le reste des composants de l'essai étant identiques, on peut en conclure que ces deux suppresseurs de FRET ont agi sélectivement sur le chélate de terbium.

**Exemple** 7 : **Utilisation d'un composé chimique, l'acide urique comme agent suppresseur du FRET entre un donneur et un accepteur.**

Dans cet exemple, il est démontré que l'acide urique est capable de supprimer sélectivement le FRET existant entre un donneur de type complexe de terre rare et un accepteur.

### *Protocole*

Deux expériences identiques visant à détecter une protéine *GST-biotine* ont été conduites. Dans chacune des expériences, un complexe de terre rare particulier a été utilisé comme donneur de FRET : soit le cryptate tris-bipyridine *TBP* soit un cryptate pyridine-bis(bipyridine) *PBP4COOH* de formule :

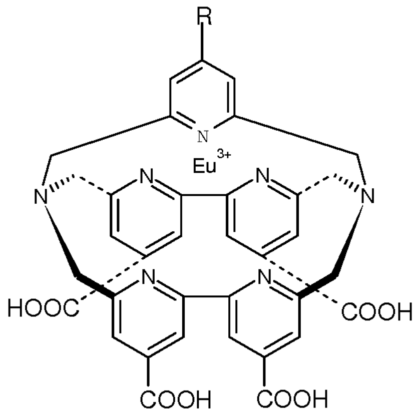

R est un groupe réactionnel classique permettant le couplage du cryptate avec la substance à marquer.

La protéine GST biotinylée a été distribuée dans une plaque de 96 puits. Sa présence a été détectée par un FRET entre le donneur (le TBP ou PBP4COOH) (porté par l'anticorps anti-GST) et l'accepteur d2 (porté par la streptavidine), après incubation pendant 16h. à température ambiante en présence ou en absence de 60μM d'acide urique. On effectue une mesure de fluorescence en temps résolu (td = 50μs, tg = 400μs) à 620nm et 665nm (respectivement E620 et E665) sur un appareil Rubystar® (BMG Labtech). A partir des valeurs d'intensité de fluorescence, E620 et E665, on calcule le rapport d'intensité E665/E620 dénommé ratio que l'on multiplie par 10 000 pour plus de commodité. Les valeurs de delta F sont calculées par rapport au ratio mesuré dans un puits négatif ne contenant pas la GST biotinylée (cf G. Mathis, Clin. Chem. 39 (1993) 1953 et la demande WO92/13264). Afin de comparer les deltas F obtenus dans les différents milieux pour un même donneur, ceux-ci sont normalisés par rapport au delta F obtenu en tampon (100%).

*Concentrations finales :*

***GST biotnylée*** 1,25nM; ***anti-GST*** 2nM; **Streptavidine** 5nM;
***Acide Urique*** 60μM

*Volume réactionnel final :* 200μl
*Tampon :* phosphate 100mM pH 7,0, BSA 0,1%

### *Résultats*

La figure 12 illustre les résultats obtenus dans les deux expériences. L'ajout de 60μM d'acide urique ne modifie pas le signal de FRET (delta F normalisé) obtenu avec le donneur PBP4COOH en présence de GST biotinylée. A l'inverse, l'acide urique agit comme suppresseur de FRET (signal de FRET inhibé de plus de 85%) si l'on utilise le TBP comme donneur pour détecter la GST biotinylée. Le reste des composants de l'essai étant identiques, on peut en conclure que ce suppresseur de FRET agit sélectivement sur le cryptate d'europium TBP.

### Exemple 8 : Détection multiplexée de la production intracellulaire de seconds messagers suite à la stimulation d'un GPCR par un agoniste.

Certains récepteurs couplés à des protéines G (GPCR), comme le récepteur muscarinique M1, peuvent être couplés à différentes voies de signalisation cellulaire pouvant aboutir à la production de seconds messagers spécifiques comme le cAMP pour la voie de signalisation liée aux les protéines Gas ou l'IP3 pour la voie de signalisation liée aux protéines Gaq.

L'activation du récepteur M1 par un agoniste spécifique pourra ainsi induire une augmentation de la concentration intracellulaire de cAMP et d'IP3. L'IP3 présentant une durée de vie intracellulaire très courte, la détection de sa production intracellulaire se fera de manière indirecte en détectant son métabolite principal l'IP1 (Trinquet et al, « D-myo-inositol 1-phosphate as a surrogate of D-myo-inositol 1,4,5-tris phosphate to monitor G protein-coupled receptor

activation » Analytical Biochemistry (2006) 358, 126-135)

Cet exemple décrit la détection multiplexée dans un même puits de microplaque de l'augmentation de la concentration intracellulaire d'IP1 et de cAMP suite à l'activation du récepteur M1 par des doses croissantes d'acétylcholine.

Dans une plaque blanche de 384 puits (Greiner, CellStar), on distribue dans chaque puits 40000 cellules CHO-K1 exprimant le récepteur muscarinique M1 préalablement diluées dans un milieu de culture RPMI contenant 10% de sérum de veau foetal et un cocktail d'antibiotiques (volume 50μl).

La plaque est mise à incuber 20h à 37°C dans une atmosphère contenant 5% CO2.

Après aspiration du milieu de culture et lavage des cellules par 50μl de tampon KREBS/HEPES, les cellules sont stimulées 1 heure à 37°C par 10μl d'une solution contenant des concentrations croissantes d'acétylcholine préalablement diluée dans un tampon KREBS contenant 25mM de LiCl, 1mM d'IBMX et 1% DMSO.

On ajoute ensuite dans chaque puits 5μl d'une solution anticorps anti IP1 marqué au cryptate d'europium trisbipyridine (anti IP1-TBP) à 4nM et 5μl d'une solution contenant 3nM d'un analogue de l'IP1 marqué avec l'accepteur d2 (IP1-d2) et 12nM d'un analogue du cAMP marqué également avec l'accepteur d2. Les solutions contenant les anticorps et les analogues marqués ont été préparées en diluant ces molécules dans un tampon HEPES 0,1M pH=7 + 0,4M KF, 1% Triton X100 et 0,1% BSA. L'ajout de ces réactifs permet la lyse cellulaire et la détection de la production intracellulaire d'IP1.

Après une heure d'incubation à température ambiante, la détection de la production d'IP1 par les cellules est réalisée en mesurant la plaque sur un lecteur Rubystar (BMG Labtech). Chaque puits est excité à 337nm par un laser à azote, les fluorescences émises à 620nm par le TBP et à 665nm par l'IP1-d2 suite au processus de transfert d'énergie non radiatif (FRET) entre l'anti IP1-TBP et l'IP1-d2 sont mesurées en temps résolu (délai=50μs ; temps d'intégration=400μs). Le ratio de la fluorescence à 665nm sur la fluorescence à 620nm est ensuite calculé pour chaque point en utilisant la formule suivante :

$$\text{Ratio} = (\text{Fluorescence à } 665\text{nm} / \text{Fluorescence à } 620\text{nm}) \times 10000$$

Comme le montre la figure 13, plus la production d'IP1 intracellulaire augmente suite à la stimulation du récepteur M1 par l'acétylcholine, plus le ratio HTRF diminue. En effet l'IP1 produit par les cellules entre en compétition avec l'IP1-d2 pour lier l'anti IP1-TBP et diminue ainsi le signal de FRET.

On ajoute ensuite dans chaque puits de la microplaque 5μl d'une solution contenant 0,92nM d'un anticorps anti cAMP marqué au cryptate d'europium 5COOH (anti cAMP-TBP5COOH) et 0,12mg/ml d'un anticorps monoclonal anti-TBP (en tant qu'agent suppresseur de signal de FRET) préalablement dilués dans un tampon HEPES 0,1M pH 7+ 0,4M KF + 0,1% BSA. L'ajout de ces réactifs va permettre la détection de la production intracellulaire de cAMP.

Après une heure d'incubation à température ambiante, la détection de la production intracellulaire de cAMP est réalisée en mesurant de nouveau la plaque sur un lecteur Rubystar (BMG Labtech). Chaque puits est excité à 337nm par un laser à azote, les fluorescences émises à 620nm par le TBP5COOH et à 665nm par le cAMP-d2 suite au processus de FRET entre l'anti cAMP-TBP5COOH et le cAMP-d2 sont mesurées en temps résolu (délai=50μs ; temps d'intégration=400μs). Le ratio de la fluorescence à 665nm sur la fluorescence à 620nm est ensuite calculé comme décrit précédemment.

La réaction de l'anticorps monoclonal anti-TBP avec le TBP supprime le signal de FRET mesuré préalablement entre l'anti IP1-TBP et l'IP1-d2. Seul le signal de FRET existant entre l'anti cAMP-TBP5COOH et le cAMP-d2 est détecté lors de cette deuxième lecture de la microplaque.

Comme le montre la figure 14, plus la production de cAMP intracellulaire augmente suite à la stimulation du récepteur M1 par l'acétylcholine, plus le ratio HTRF diminue. En effet le cAMP produit par les cellules entre en compétition avec le cAMP-d2 pour lier l'anti cAMP-TBP5COOH et diminue ainsi le signal de FRET.

**Références bibliographiques** :

Lynch BA, Minor C, Loiacono KA, van Schravendijk MR, Ram MK, Sundaramoorthi R, Adams SE, Phillips T, Holt D, Rickles RJ, MacNeil IA Simultaneous assay of Src SH3 and SH2 domain binding using different wavelength fluorescence polarization probes. Anal Biochem. 1999 275(1):62-73

Blommel P, Hanson GT, Vogel KW Multiplexing fluorescence polarization assays to increase information content per screen: applications for screening steroid hormone receptors. J Biomol Screen. 2004 9(4):294-302

Swartzman et Al. An homogeneous and multiplexed immunoassay for high-throughput screening using fluorometric microvolume assay technology. Anal. Biochem. (1999), 271, 143-151

Chan-Hui PY, Stephens K, Warnock RA, Singh S Applications of eTag trade mark assay platform to systems biology approaches in molecular oncology and toxicology studies. Clin Immunol. 2004 111(2):162-74

Tian H, Cao L, Tan Y, Williams S, Chen L, Matray T, Chenna A, Moore S, Hernandez V, Xiao V, Tang M, Singh S Multiplex mRNA assay using electrophoretic tags for high-throughput gene expression analysis. Nucleic Acids Res. 2004 32(16):e126

Bradford JA, Buller G, Suter M, Ignatius M, Beechem JM Fluorescence-intensity multiplexing: simultaneous seven-marker, two-color immunophenotyping using flow cytometry. Cytometry A. 2004 61(2):142-52

Kurner JM, Klimant I, Krause C, Pringsheim E, Wolfbeis OS A new type of phosphorescent nanospheres for use in advanced time-resolved multiplexed bioassays. Anal Biochem. 2001 297(1):32-41

Tong AK, Li Z, Jones GS, Russo JJ, Ju J Combinatorial fluorescence energy transfer tags for multiplex biological assays. Nat Biotechnol. 2001 19(8):756-9

Tyagi S, Marras SA, Kramer FR Wavelength-shifting molecular beacons. Nat Biotechnol. 2000 18(11):1191-6

Watt et Al. Immunochemistry 1997, 14,533-541

Babendure JR, Adams SR, Tsien RY Aptamers switch on fluorescence of triphenylmethane dyes. J Am Chem Soc. 2003 125(48):14716-7

**Revendications**

**1.** Procédé de détection de deux évènements biologiques dans un milieu réactionnel, comprenant les étapes suivantes :

- introduction dans le milieu réactionnel de 2 couples de conjugués fluorescents partenaires de FRET, au moins l'un des conjugués fluorescents comprenant un chélate ou un cryptate de terre rare, chaque couple étant spécifique d'un des évènements biologiques étudiés, et la longueur d'onde à laquelle les conjugués fluorescents donneurs sont excités étant la même pour tous les couples, et la longueur d'onde à laquelle sont mesurés les signaux émis par les conjugués fluorescents accepteurs étant la même pour tous les couples ;
- introduction d'un agent suppresseur de signal de FRET capable de supprimer sélectivement le FRET entre deux partenaires de FRET, ledit agent étant un composé qui provoque une diminution de signal de FRET d'au moins 70% par rapport au signal mesuré en l'absence de ce composé ;
- mesure de manière séquentielle des différents signaux de FRET émis par le milieu réactionnel ; ledit agent suppresseur de signal de FRET étant :
    - soit, un anticorps anti-chélate de terre rare ou anti-cryptate de terre rare ;
    - soit, un ion rentrant en compétition avec la terre rare pour la liaison avec le chélate ou un agent complexant les métaux ;
    - soit, l'acide urique.

**2.** Procédé de détection de deux évènements biologiques dans un milieu réactionnel selon la revendication 1, comprenant les étapes suivantes :

(i) mise en contact du milieu réactionnel avec un premier couple de conjugués fluorescents partenaires de FRET spécifiques d'un premier évènement biologique ;
(ii) excitation du milieu réactionnel à la longueur d'onde $\lambda$1 ;
(iii) mesure du signal de FRET émis par le milieu réactionnel à la longueur d'onde À2 et correspondant au premier événement biologique ;
(iv) introduction dans le milieu réactionnel d'un agent suppresseur du signal de FRET spécifique du couple de partenaires de FRET mis en contact dans le milieu, ledit agent suppresseur du signal de FRET étant tel que défini à la revendication 1 ;

(v) mise en contact du milieu réactionnel avec un couple de conjugués fluorescents partenaires de FRET spécifiques du second évènement biologique ;
(vi) excitation du milieu réactionnel à la longueur d'onde λ1 ;
(vii) mesure du signal de FRET émis par le milieu réactionnel à la longueur d'onde À2, correspondant à l'événement biologique dont est spécifique le second couple de partenaires de FRET mis en contact dans le milieu ;

les conjugués fluorescents partenaires de FRET comprenant un fluorophore donneur et un fluorophore accepteur, λ1 étant la longueur d'onde d'excitation des fluorophores donneurs et À2 étant la longueur d'onde d'émission des fluorophores accepteurs.

**3.** Procédé de détection de deux évènements biologiques dans un milieu réactionnel selon la revendication 2, comprenant les étapes suivantes :

(i) mise en contact du milieu réactionnel avec deux couples de conjugués fluorescents partenaires de FRET spécifiques des deux évènements biologiques ;
(ii) excitation du milieu réactionnel à la longueur d'onde λ1 ;
(iii) mesure du signal de FRET émis par le milieu réactionnel et correspondant aux deux événements biologiques étudiés ;
(iv) introduction dans le milieu réactionnel d'un agent suppresseur du signal de FRET spécifique d'un desdits couples de conjugués fluorescents partenaires de FRET, ledit agent suppresseur du signal de FRET étant tel que défini à la revendication 1 ;
(v) excitation du milieu réactionnel à la longueur d'onde λ1 ;
(vi) mesure du signal de FRET émis par le milieu réactionnel, correspondant à tous les évènements réactionnels moins celui dont le signal a été bloqué dans une étape précédente ;
(vii) Détermination des valeurs de signal de FRET pour chacun des deux événements biologiques, par la résolution mathématique des deux équations obtenues dans les étapes (iii) et (vi) ;

les conjugués fluorescents partenaires de FRET comprenant un fluorophore donneur et un fluorophore accepteur, λ1 étant la longueur d'onde d'excitation des fluorophores donneurs et À2 étant la longueur d'onde d'émission des fluorophores accepteurs.

**4.** Procédé selon l'une quelconque des revendications 1 à 3, **caractérisé en ce qu'**il comprend au moins une étape de stimulation appliquée au milieu réactionnel, cette stimulation étant de nature chimique, pharmacologique, électrique, thermique ou mécanique.

**5.** Procédé selon la revendication 4, **caractérisé en ce que** l'étape de stimulation consiste à rajouter au milieu réactionnel un agoniste, un antagoniste, un agoniste inverse ou un modulateur allostérique d'un récepteur donné.

**6.** Procédé selon l'une quelconque des revendications 1 à 5, **caractérisé en ce que** chacun des conjugués fluorescents partenaires de FRET spécifique d'un événement biologique comprend un fluorophore lié de manière covalente à une sonde, cette sonde étant spécifique dudit événement biologique, et ladite sonde étant constituée d'une molécule impliquée dans cet événement, ou bien comprend un domaine de liaison à une molécule impliquée dans cet évènement.

**7.** Procédé selon la revendication 6, **caractérisé en ce que** la sonde est une molécule impliquée dans un événement biologique, choisie parmi : une protéine, un peptide, une enzyme, un substrat d'enzyme, un messager intracellulaire, un nucléotide cyclique, un phospholipide, un facteur de transcription, un composé comportant un cycle inositol, un acide nucléique, un oligonucléotide.

**8.** Procédé selon la revendication 6, **caractérisé en ce que** la sonde comprend un domaine de liaison à une molécule impliquée dans un événement biologique, et est choisie parmi : un anticorps ou un fragment d'anticorps ; un anticorps ou fragment d'anticorps anti-étiquette (anti-tag) reconnaissant une étiquette (tag) greffée sur la molécule impliquée dans ledit évènement biologique; un aptamère ; un peptide ; une protéine ; un acide nucléique simple-brin pouvant s'hybrider avec un acide nucléique de séquence complémentaire greffé sur la molécule impliquée dans ledit évènement biologique.

**9.** Procédé selon la revendication 6, **caractérisé en ce que** les fluorophores partenaires de FRET sont choisis dans le groupe suivant : les protéines luminescentes, les protéines fluorescentes extraites de coraux, les phycobiliprotéines, les molécules organiques luminescentes ; les cryptates de terre rare, les chélates de terre rare ; les particules inorganiques luminescentes.

**10.** Procédé selon l'une quelconque des revendications 1 à 9, **caractérisé en ce que** l'un des conjugués fluorescents comporte un chélate ou un cryptate d'europium tris-bipyridine, et **en ce que** l'agent suppresseur du signal de FRET est un anticorps anti-cryptate d'europium tris-bipyridine.

**11.** Procédé selon l'une quelconque des revendications 1 à 9, **caractérisé en ce qu'**au moins un des couples de conjugués fluorescents partenaires de FRET comprend un chélate de terre rare, et **en ce que** l'ion rentrant en compétition avec la terre rare pour la liaison avec le chélate est l'ion $Mn^{2+}$.

**12.** Procédé selon l'une quelconque des revendications 1 à 9, **caractérisé en ce qu'**au moins un des couples de conjugués fluorescents partenaires de FRET comprend un chélate de terre rare, et **en ce que** l'agent complexant les métaux est l'EDTA.

**13.** Procédé selon l'une quelconque des revendications 1 à 12, **caractérisé en ce que** l'événement biologique est choisi parmi : une réaction enzymatique, la présence ou la variation de concentration d'un analyte dans le milieu réactionnel, le rapprochement de deux molécules dans le milieu réactionnel, les modifications de conformations tridimensionnelles de molécules.

**14.** Agent suppresseur du signal FRET entre 2 fluorophores partenaires de FRET dont l'un est un cryptate d'europium tris-bipyridine, **caractérisé en ce que** ledit agent possède un domaine de liaison avec ce cryptate d'europium tris-bipyridine, **en ce qu'**il provoque une diminution d'au moins 70% du signal de FRET par rapport au signal émis en l'absence dudit agent, et **en ce qu'**il est un anticorps anti-cryptate d'europium tris-bipyridine.

**15.** Nécessaire de composants, **caractérisé en ce qu'**il comprend les éléments suivants, fournis ensemble ou séparément :

- deux couples de conjugués fluorescents partenaires de FRET spécifiques de deux événements biologiques,
- un agent suppresseur du signal FRET tel que défini à la revendication 1.

**16.** Utilisation du procédé selon l'une quelconque des revendications 1 à 3 pour le criblage de composés biologiquement actifs.

## Patentansprüche

**1.** Verfahren zur Erfassung von zwei biologischen Ereignissen in einem Reaktionsmedium, umfassend die folgenden Schritte:

- Einbringen von zwei Paaren von fluoreszierenden FRET-Partner-Konjugaten in das Reaktionsmedium, wobei wenigstens eines der fluoreszierenden Konjugate ein Chelat oder ein Kryptat von Seltene Erde umfasst, wobei jedes Paar für eines der untersuchten biologischen Ereignisse spezifisch ist, und wobei die Wellenlänge, bei der die fluoreszierenden Donor-Konjugate angeregt werden, für alle Paare die gleiche ist, und wobei die Wellenlänge, bei der die durch die fluoreszierenden Akzeptor-Konjugate ausgesandten Signale gemessen werden, für alle Paare die gleiche ist,
- Einbringen eines Mittels zur Unterdrückung des FRET-Signals, das geeignet ist, den FRET zwischen zwei FRET-Partnern selektiv zu unterdrücken, wobei das Mittel eine Verbindung ist, die eine FRET-Signal-Verringerung um wenigstens 70 % gegenüber dem bei Abwesenheit dieser Verbindung gemessenen Signal bewirkt,
- sequentielles Messen der verschiedenen durch das Reaktionsmedium ausgesandten FRET-Signale,

wobei das Mittel zur Unterdrückung des FRET-Signals

- entweder ein anti-Seltenerd-Chelat- oder anti-Seltenerd-Kryptat-Antikörper
- oder ein Ion, das mit der Seltenerde für die Bindung mit dem Chelat konkurriert oder ein die Metalle komplexierenden Mittel,
- oder Harnsäure

ist.

2. Verfahren zur Erfassung von zwei biologischen Ereignissen in einem Reaktionsmedium nach Anspruch 1, umfassend die folgenden Schritte:

(i) Inkontaktbringen des Reaktionsmediums mit einem ersten Paar von fluoreszierenden FRET-Partner-Konjugaten, die für ein erstes biologisches Ereignis spezifisch sind,
(ii) Anregen des Reaktionsmediums bei der Wellenlänge λ1,
(iii) Messen des FRET-Signals, das durch das Reaktionsmedium bei der Wellenlänge λ2 ausgesandt wird und dem ersten biologischen Ereignis entspricht,
(iv) Einbringen eines Mittels zur Unterdrückung des FRET-Signals, welches für das in dem Medium in Kontakt gebrachte Paar von FRET-Partnern spezifisch ist, in das Reaktionsmedium, wobei das Mittel zur Unterdrückung des FRET-Signals so ist wie in Anspruch 1 definiert,
(v) Inkontaktbringen des Reaktionsmediums mit einem Paar von fluoreszierenden FRET-Partner-Konjugaten, die für das zweite biologische Ereignis spezifisch sind,
(vi) Anregen des Reaktionsmediums bei der Wellenlänge λ1,
(vii) Messen des durch das Reaktionsmedium bei der Wellenlänge λ2 ausgesandten FRET-Signals, das dem biologischen Ereignis entspricht, für das das in dem Medium in Kontakt gebrachte zweite Paar von FRET-Partnern spezifisch ist,

wobei die fluoreszierenden FRET-Partner-Konjugate einen Donor-Fluorophor und einen Akzeptor-Fluorophor umfassen, wobei λ1 die Anregungswellenlänge der Donor-Fluorophore ist und λ2 die Emissionswellenlänge der Akzeptor-Fluorophore ist.

3. Verfahren zur Erfassung von zwei biologischen Ereignissen in einem Reaktionsmedium nach Anspruch 2, umfassend die folgenden Schritte:

(i) Inkontaktbringen des Reaktionsmediums mit zwei Paaren von fluoreszierenden FRET-Partner-Konjugaten, die für die beiden biologischen Ereignisse spezifisch sind,
(ii) Anregen des Reaktionsmediums bei der Wellenlänge λ1,
(iii) Messen des FRET-Signals, das durch das Reaktionsmedium ausgesandt wird und den beiden untersuchten biologischen Ereignissen entspricht,
(iv) Einbringen eines Mittels zur Unterdrückung des FRET-Signals, welches für eines der Paare von fluoreszierenden FRET-Partner-Konjugaten spezifisch ist, in das Reaktionsmedium, wobei das Mittel zur Unterdrückung des FRET-Signals so ist wie in Anspruch 1 definiert,
(v) Anregen des Reaktionsmediums bei der Wellenlänge λ1,
(vi) Messen des durch das Reaktionsmedium ausgesandten FRET-Signals, das allen Reaktionsereignissen minus demjenigen, dessen Signal in einem vorhergehenden Schritt blockiert worden ist, entspricht,
(vii) Bestimmen der FRET-Signal-Werte für jedes der beiden biologischen Ereignisse durch mathematische Auflösung der beiden in den Schritten (iii) und (vi) erhaltenen Gleichungen,

wobei die fluoreszierenden FRET-Partner-Konjugate einen Donor-Fluorophor und einen Akzeptor-Fluorophor umfassen, wobei λ1 die Anregungswellenlänge der Donor-Fluorophore ist und λ2 die Emissionswellenlänge der Akzeptor-Fluorophore ist.

4. Verfahren nach einem der Ansprüche 1 bis 3, **dadurch gekennzeichnet, dass** es wenigstens einen Stimulationsschritt, der auf das Reaktionsmedium angewandt wird, umfasst, wobei diese Stimulation chemischer, pharmakologischer, elektrischer, thermischer oder mechanischer Art ist.

5. Verfahren nach Anspruch 4, **dadurch gekennzeichnet, dass** der Stimulationsschritt darin besteht, dem Reaktionsmedium einen Agonisten, einen Antagonisten, einen inversen Agonisten oder einen allosterischen Modulator eines gegebenen Rezeptors hinzuzufügen.

6. Verfahren nach einem der Ansprüche 1 bis 5, **dadurch gekennzeichnet, dass** ein jedes der fluoreszierenden FRET-Partner-Konjugate, das für ein biologisches Ereignis spezifisch ist, einen Fluorophor umfasst, der an eine Sonde kovalent gebunden ist, wobei diese Sonde für das biologische Ereignis spezifisch ist, und wobei die Sonde von einem an diesem Ereignis beteiligten Molekül gebildet ist, oder aber eine Domäne zur Bindung an ein an diesem Ereignis beteiligtes Molekül umfasst.

7. Verfahren nach Anspruch 6, **dadurch gekennzeichnet, dass** die Sonde ein an einem biologischen Ereignis beteiligtes Molekül ist, ausgewählt aus: einem Protein, einem Peptid, einem Enzym, einem Enzymsubstrat, einem intrazellulären Messenger, einem zyklischen Nukleotid, einem Phospholipid, einem Transkriptionsfaktor, einer Verbindung mit einem Inositolring, einer Nukleinsäure, einem Oligonukleotid.

8. Verfahren nach Anspruch 6, **dadurch gekennzeichnet, dass** die Sonde eine Domäne zur Bindung an ein an einem biologischen Ereignis beteiligtes Molekül umfasst und ausgewählt ist aus: einem Antikörper oder einem Antikörperfragment; einem anti-tag-Antikörper oder -Antikörperfragment, der/das einen auf das an dem biologischen Ereignis beteiligte Molekül gepfropften Tag erkennt, einem Aptamer, einem Peptid, einem Protein; einer einsträngigen Nukleinsäure, die mit einer Nukleinsäure komplementärer Sequenz, welche auf das an dem biologischen Ereignis beteiligte Molekül gepfropft ist, hybridisieren kann.

9. Verfahren nach Anspruch 6, **dadurch gekennzeichnet, dass** die FRET-Partner-Fluorophore aus der folgenden Gruppe ausgewählt sind: den lumineszierenden Proteinen, den aus Korallen extrahierten fluoreszierenden Proteinen, den Phycobiliproteinen, den lumineszierenden organischen Molekülen, den Seltenerd-Kryptaten, den Seltenerd-Chelaten, den lumineszierenden anorganischen Partikeln.

10. Verfahren nach einem der Ansprüche 1 bis 9, **dadurch gekennzeichnet, dass** eines der fluoreszierenden Konjugate ein Europium tris-Bipyridin Chelat oder Kryptat umfasst und dass das Mittel zur Unterdrückung des FRET-Signals ein anti-Europium tris-Bipyridin Kryptat-Antikörper ist.

11. Verfahren nach einem der Ansprüche 1 bis 9, **dadurch gekennzeichnet, dass** wenigstens eines der Paare von fluoreszierenden FRET-Partner-Konjugaten ein Seltenerd-Chelat umfasst und dass das Ion, das mit der Seltenerde für die Bindung mit dem Chelat konkurriert, das Ion $Mn^{2+}$ ist.

12. Verfahren nach einem der Ansprüche 1 bis 9, **dadurch gekennzeichnet, dass** wenigstens eines der Paare von fluoreszierenden FRET-Partner-Konjugaten ein Seltenerd-Chelat umfasst und dass das die Metalle komplexierende Mittel EDTA ist.

13. Verfahren nach einem der Ansprüche 1 bis 12, **dadurch gekennzeichnet, dass** das biologische Ereignis ausgewählt ist aus: einer enzymatischen Reaktion, dem Vorliegen oder der Variation der Konzentration eines Analyten in dem Reaktionsmedium, der Annäherung von zwei Molekülen in dem Reaktionsmedium, den Änderungen von dreidimensionalen Molekülgestaltungen.

14. Mittel zur Unterdrückung des FRET-Signals zwischen zwei FRET-Partner-Fluorophoren, von denen einer ein Europium tris-Bipyridin Kryptat ist, **dadurch gekennzeichnet, dass** das Mittel eine Bindungsdomäne mit diesem Europium tris-Bipyridin Kryptat besitzt, dass es eine Verringerung um wenigstens 70 % des FRET-Signals gegenüber dem bei Abwesenheit des Mittels ausgesandten Signal bewirkt, und dass es ein anti-Europium tris-Bipyridin Kryptat-Antikörper ist.

15. Komponentensatz, **dadurch gekennzeichnet, dass** er die folgenden Elemente, die gemeinsam oder getrennt bereitgestellt werden, umfasst:

- zwei Paare von fluoreszierenden FRET-Partner-Konjugaten, die für zwei biologische Ereignisse spezifisch sind,
- ein Mittel zur Unterdrückung des FRET-Signals wie es in Anspruch 1 definiert ist.

16. Verwendung des Verfahrens nach einem der Ansprüche 1 bis 3 für das Screening von biologisch aktiven Verbindungen.

## Claims

1. A method for detecting two biological events in a reaction medium, comprising the following steps:

- introducing, into the reaction medium, 2 pairs of fluorescent FRET partner conjugates, at least one of the fluorescent conjugates comprising a rare earth chelate or cryptate, each pair being specific for one of the biological events studied, the wavelength at which the fluorescent donor conjugates are excited being the same for all the pairs, and the wavelength at which the signals emitted by the fluorescent acceptor conjugates are

measured being the same for all the pairs;
- introducing a FRET signal killer agent capable of selectively suppressing the FRET between two FRET partners, said killer agent being a compound which causes a decrease of the FRET signal of at least 70% compared to the signal measured in the absence of said compound;
- sequentially measuring the various FRET signals emitted by the reaction medium;

the FRET signal killer agent being:

- either an anti-rare earth chelate antibody or an anti-rare earth cryptate antibody;
- or an ion which competes with the rare earth for the binding with the chelate, or an agent which complexes metals;
- or uric acid.

**2.** The method for detecting two biological events in a reaction medium according to claim 1, comprising the following steps:

(i) bringing the reaction medium into contact with a first pair of fluorescent FRET partner conjugates specific for a first biological event;
(ii) exciting the reaction medium at the wavelength λ1;
(iii) measuring the FRET signal emitted by the reaction medium at the wavelength λ2 and corresponding to the first biological event;
(iv) introducing into the reaction medium a FRET signal killer agent specific for the pair of FRET partners brought into contact in the medium, said FRET signal killer agent being as defined in claim 1;
(v)bringing the reaction medium into contact with a pair of fluorescent FRET partner conjugates specific for the second biological;
(vi) exciting the reaction medium at the wavelength λ1;
(vii) measuring the FRET signal emitted by the reaction medium at the wavelength λ2, corresponding to the biological event for which the second pair of FRET partners brought into contact in the medium is specific;

the fluorescent FRET partner conjugates comprising a donor fluorophore and an acceptor fluorophore, λ1 being the excitation wavelength of the donor fluorophores and λ2 being the emission wavelength of the acceptor fluorophores.

**3.** The method for detecting two biological events in a reaction medium according to claim 2, comprising the following steps:

(i) bringing the reaction medium into contact with two pairs of fluorescent FRET partner conjugates specific for the two biological events;
(ii) exciting the reaction medium at the wavelength λ1;
(iii) measuring the FRET signal emitted by the reaction medium and corresponding to the two biological events studied;
(iv) introducing into the reaction medium a FRET signal killer agent specific for one of said pairs of fluorescent FRET partner conjugates, said FRET signal killer agent being as defined in claim 1;
(v) exciting the reaction medium at the wavelength λ1;
(vi) measuring the FRET signal emitted by the reaction medium, corresponding to all the reaction events minus that for which the signal was blocked in a preceding step;
(vii) determining the FRET signal values for each of the two biological events, by mathematical resolution of the 2 equations obtained in steps (iii) and (vi);

the fluorescent FRET partner conjugates comprising a donor fluorophore and an acceptor fluorophore, λ1 being the excitation wavelength of the donor fluorophores and λ2 being the emission wavelength of the acceptor fluorophores.

**4.** The method as claimed in any one of claims 1 to 3, **characterized in that** it comprises at least one stimulation step applied to the reaction medium, this stimulation being of chemical, pharmacological, electrical, thermal or mechanical nature.

**5.** The method as claimed in claim 4, **characterized in that** the stimulation step consists in adding, to the reaction medium, an agonist, an antagonist, an inverse agonist or an allosteric modulator of a given receptor.

**6.** The method as claimed in any one of claims 1 to 5, **characterized in that** each of the fluorescent FRET partner

conjugates specific for a biological event comprises a fluorophore covalently bound to a probe, this probe being specific for said biological event, and wherein said probe consists of a molecule involved in this event, or else comprises a domain for binding to a molecule involved in this event.

**7.** The method as claimed in claim 6, **characterized in that** the probe is a molecule involved in a biological event, chosen from: a protein, a peptide, an enzyme, an enzyme substrate, an intracellular messenger, a cyclic nucleotide, a phospholipid, a transcription factor, a compound comprising an inositol ring, a nucleic acid and an oligonucleotide.

**8.** The method as claimed in claim 6, **characterized in that** the probe comprises a domain for binding to a molecule involved in a biological event, and is chosen from: an antibody or antibody fragment; an anti-tag antibody or antibody fragment which recognizes a tag grafted on the molecule involved in said biological event; an aptamer; a peptide; a protein; a single-stranded nucleic acid which can hybridize with a nucleic acid of complementary sequence grafted onto the molecule involved in said biological event.

**9.** The method as claimed in claim 6, **characterized in that** the FRET partner fluorophores are chosen from the following group: luminescent proteins, fluorescent proteins extracted from coral, phycobiliproteins, luminescent organic molecules, rare earth cryptates, rare earth chelates and luminescent inorganic particles.

**10.** The method as claimed in any one of claims 1 to 9, **characterized in that** one of the fluorescent conjugates comprises a tris-bipyridine europium chelate or cryptate, and **in that** the FRET signal killer agent is an anti-tris-bipyridine europium cryptate antibody.

**11.** The method as claimed in any one of claims 1 to 9, **characterized in that** at least one of the pairs of fluorescent FRET partner conjugates comprises a rare earth chelate, and **in that** the ion which competes with the rare earth for the binding with the chelate is the ion $Mn^{2+}$.

**12.** The method as claimed in any one of claims 1 to 9, **characterized in that** at least one of the pairs of fluorescent FRET partner conjugates comprises a rare earth chelate, and **in that** the agent which complexes metals is EDTA.

**13.** The method as claimed in any one of claims 1 to 12, **characterized in that** the biological event is chosen from: an enzyme reaction, the presence or the variation in concentration of an analyte in the reaction medium, two molecules coming closer together in the reaction medium, and modifications to the three-dimensional conformations of molecules.

**14.** An agent for killing the FRET signal between two fluorophores which are FRET partners, one of which being a tris-bipyridine europium cryptate, **characterized in that** said agent comprises a domain for binding with said tris-bipyridine europium cryptate, **in that** it causes a decrease of the FRET signal of at least 70% compared to the signal measured in the absence of said agent, and **in that** it is an anti-tris-bipyridine europium cryptate antibody.

**15.** A kit of parts, **characterized in that** it comprises the following elements, provided together or separately:

- two pairs of fluorescent FRET partner conjugates specific for two biological events,
- a FRET signal killer agent as defined in claim 1.

**16.** The use of the method as claimed in any one of claims 1 to 3, for screening for biologically active compounds.

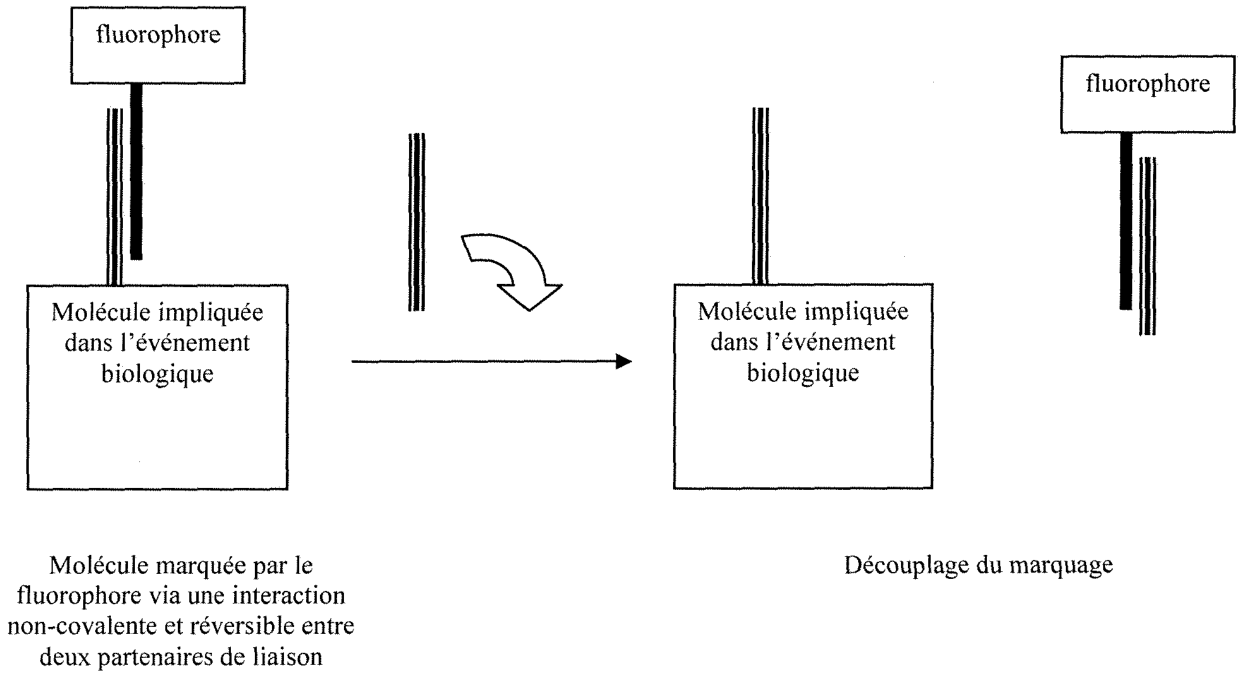
fluorophore
Molécule impliquée dans l'événement biologique
Molécule marquée par le fluorophore via une interaction non-covalente et réversible entre deux partenaires de liaison
Molécule impliquée dans l'événement biologique
fluorophore
Découplage du marquage

**Fig. 1**

*Etape I:*
*détection de la **protéine totale** par FRET $D_1$-A*
***(signal $S_T$)***

*Etape II:*
*extinction du signal $S_T$ de la protéine totale*
*par un produits FRET-killer*
*qui reconnaît spécifiquement le donneur $D_1$*
*et détection de la **protéine phosphorylée** par FRET $D_2$-A*
*(signal $S_P$)*

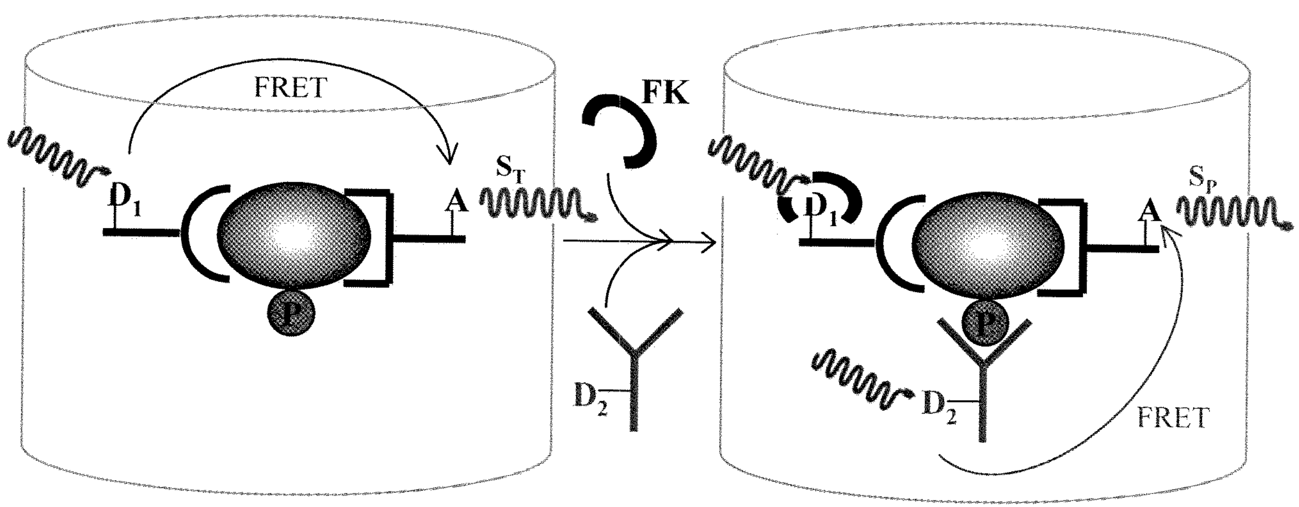


**Fig. 2**

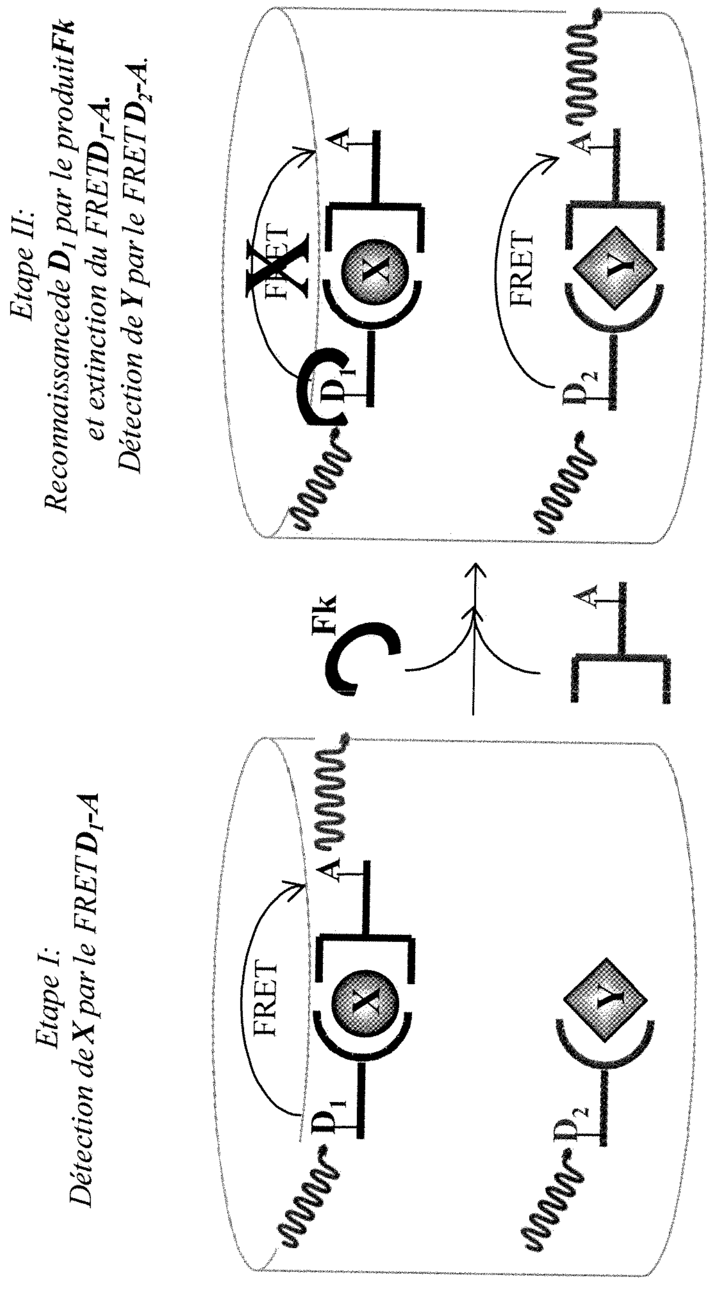
Etape I:
Détection de X par le FRET D1-A
FRET
D1
A
X
Y
D2
Fk
Etape II:
Reconnaissancede D1 par le produitFk
et extinction du FRETD1-A.
Détection de Y par le FRETD2-A.
FRET
D1
A
X
Y
D2
FRET
A

Fig. 3

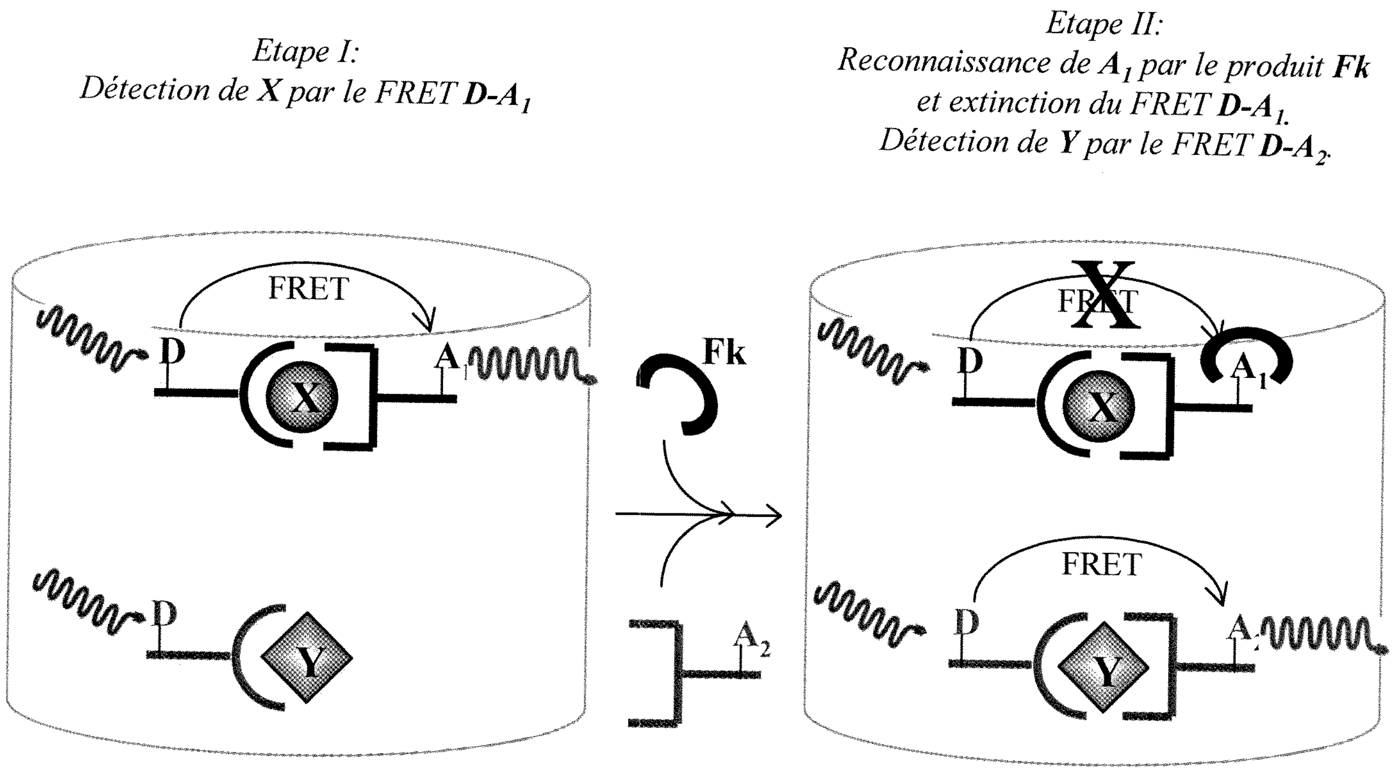
Etape I:
Détection de **X** par le FRET **D-A**$_1$
Etape II:
Reconnaissance de **A**$_1$ par le produit **Fk**
et extinction du FRET **D-A**$_1$.
Détection de **Y** par le FRET **D-A**$_2$.
FRET
D
A$_1$
X
Y
Fk
A$_2$

Fig. 4

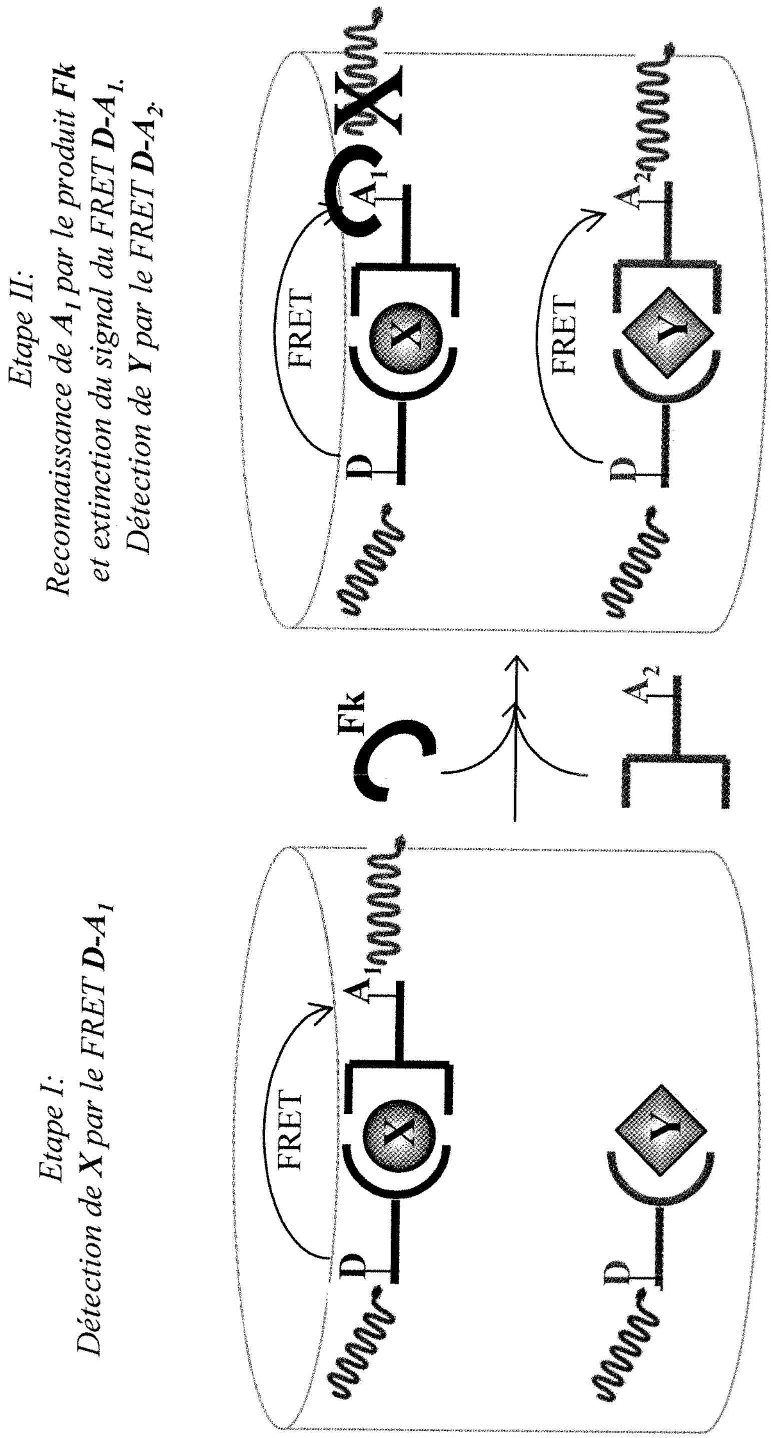
Etape I:
Détection de X par le FRET D-A1
FRET
D
X
A1
Y
D
Etape II:
Reconnaissance de A1 par le produit Fk
et extinction du signal du FRET D-A1.
Détection de Y par le FRET D-A2.
FRET
D
X
A1
FRET
D
Y
A2
Fk
A2

Fig. 5

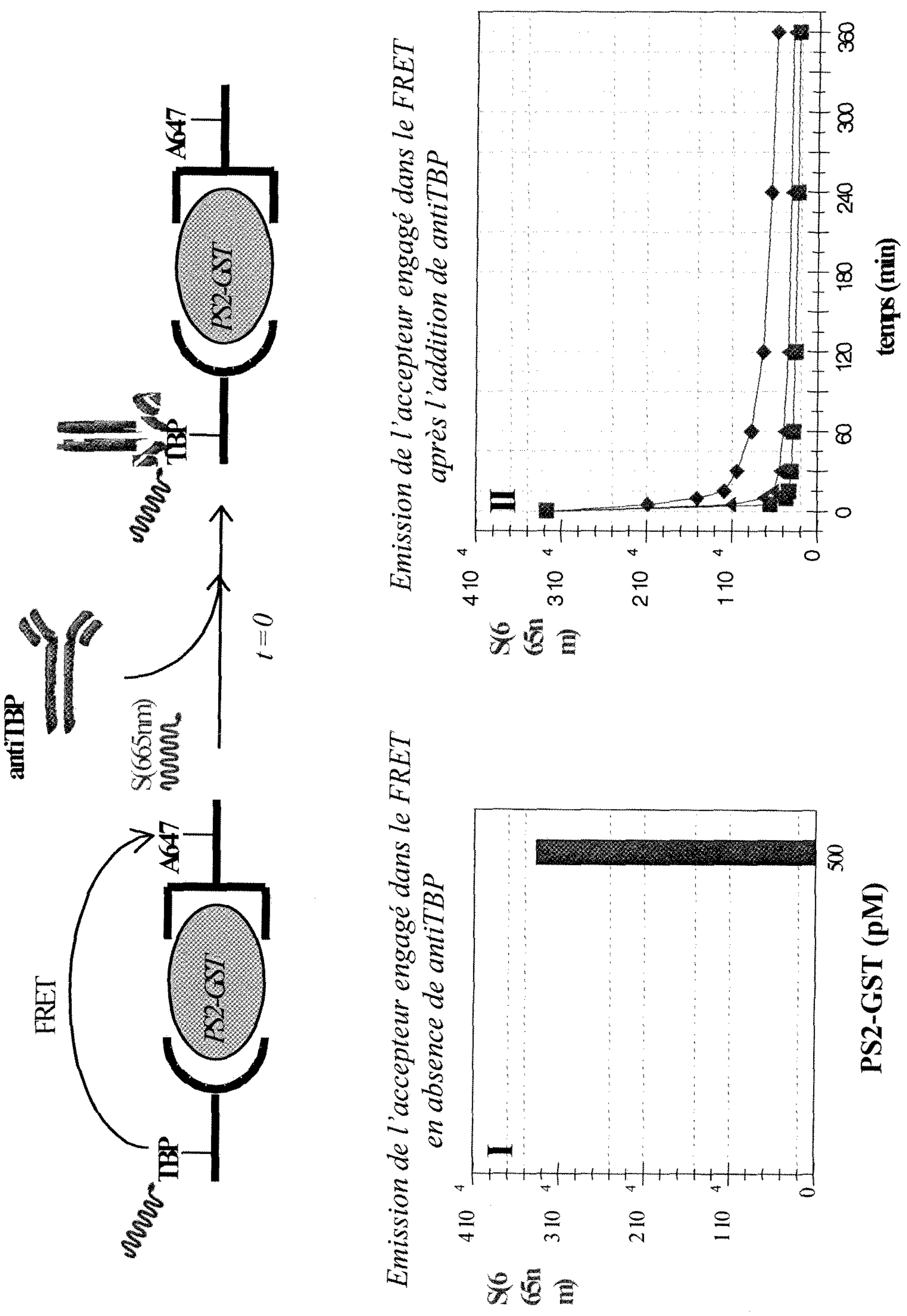
FRET
TBP
PS2-GST
A647
antiTBP
S(665nm)
t=0
Emission de l'accepteur engagé dans le FRET en absence de antiTBP
I
S(665nm)
PS2-GST (pM)
500
Emission de l'accepteur engagé dans le FRET après l'addition de antiTBP
II
temps (min)
0
60
120
180
240
300
360

Fig. 6A

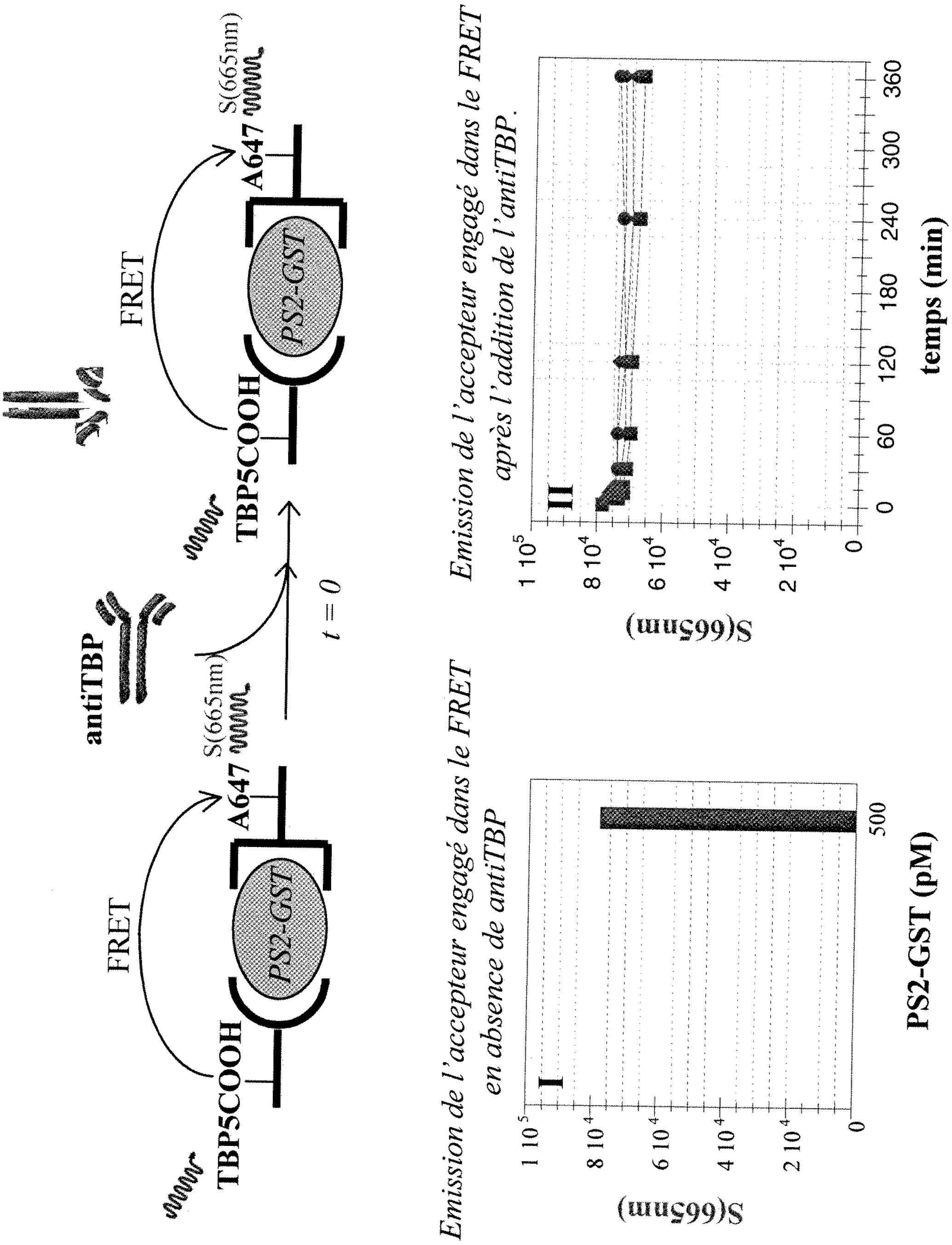
FRET
TBP5COOH
PS2-GST
A647
S(665nm)
antiTBP
t = 0
FRET
TBP5COOH
PS2-GST
A647
S(665nm)
Emission de l'accepteur engagé dans le FRET en absence de antiTBP
I
S(665nm)
1 10^5
8 10^4
6 10^4
4 10^4
2 10^4
0
500
PS2-GST (pM)
Emission de l'accepteur engagé dans le FRET après l'addition de l'antiTBP.
II
S(665nm)
1 10^5
8 10^4
6 10^4
4 10^4
2 10^4
0
0
60
120
180
240
300
360
temps (min)

Fig. 6B

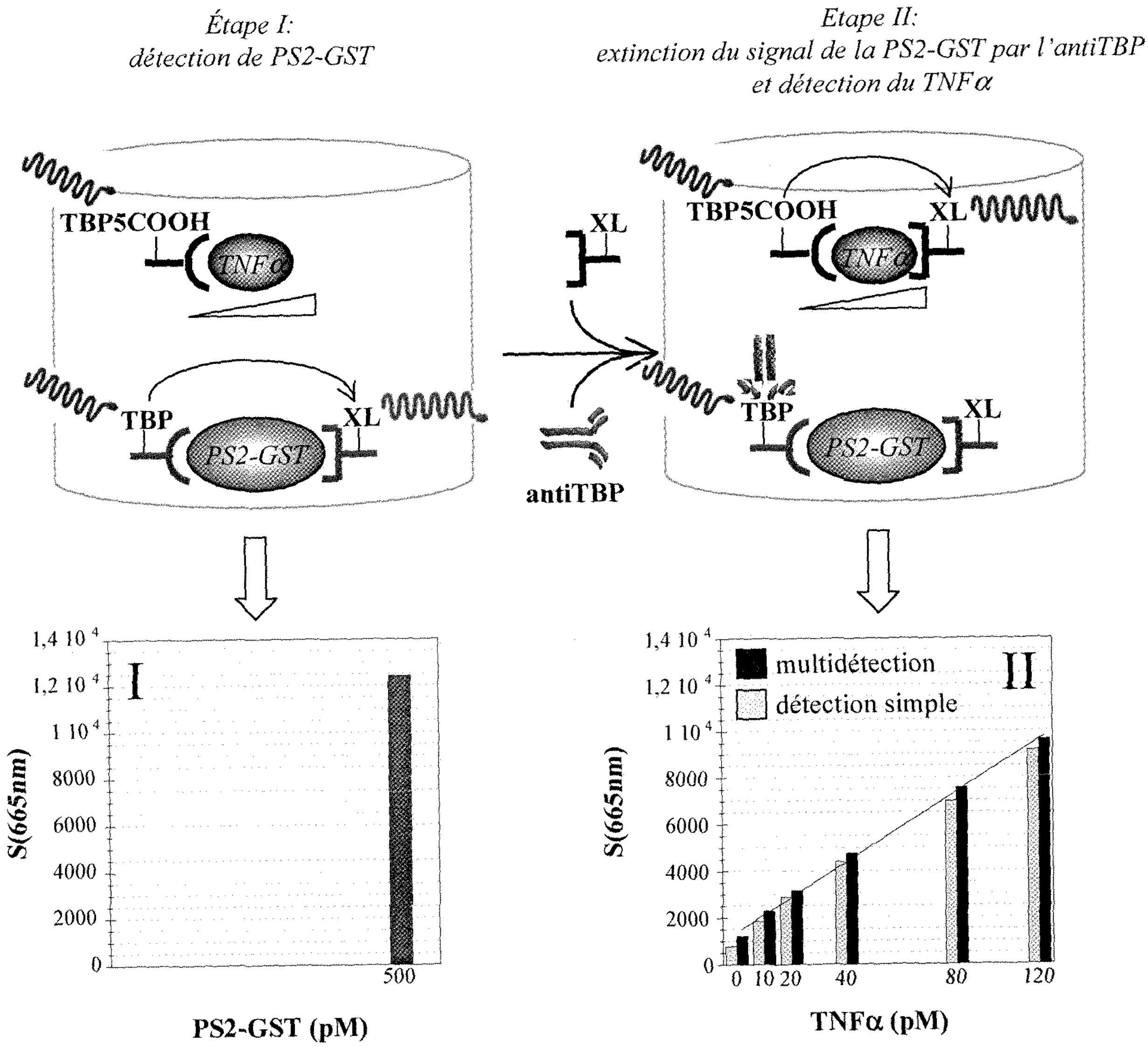
Étape I:
détection de PS2-GST
Etape II:
extinction du signal de la PS2-GST par l'antiTBP
et détection du TNFα
TBP5COOH
TNFα
TBP
PS2-GST
XL
antiTBP
1,4 10 4
1,2 10 4
1 10 4
8000
6000
4000
2000
0
S(665nm)
I
500
PS2-GST (pM)
II
multidétection
détection simple
0 10 20 40 80 120
TNFα (pM)

Fig. 7

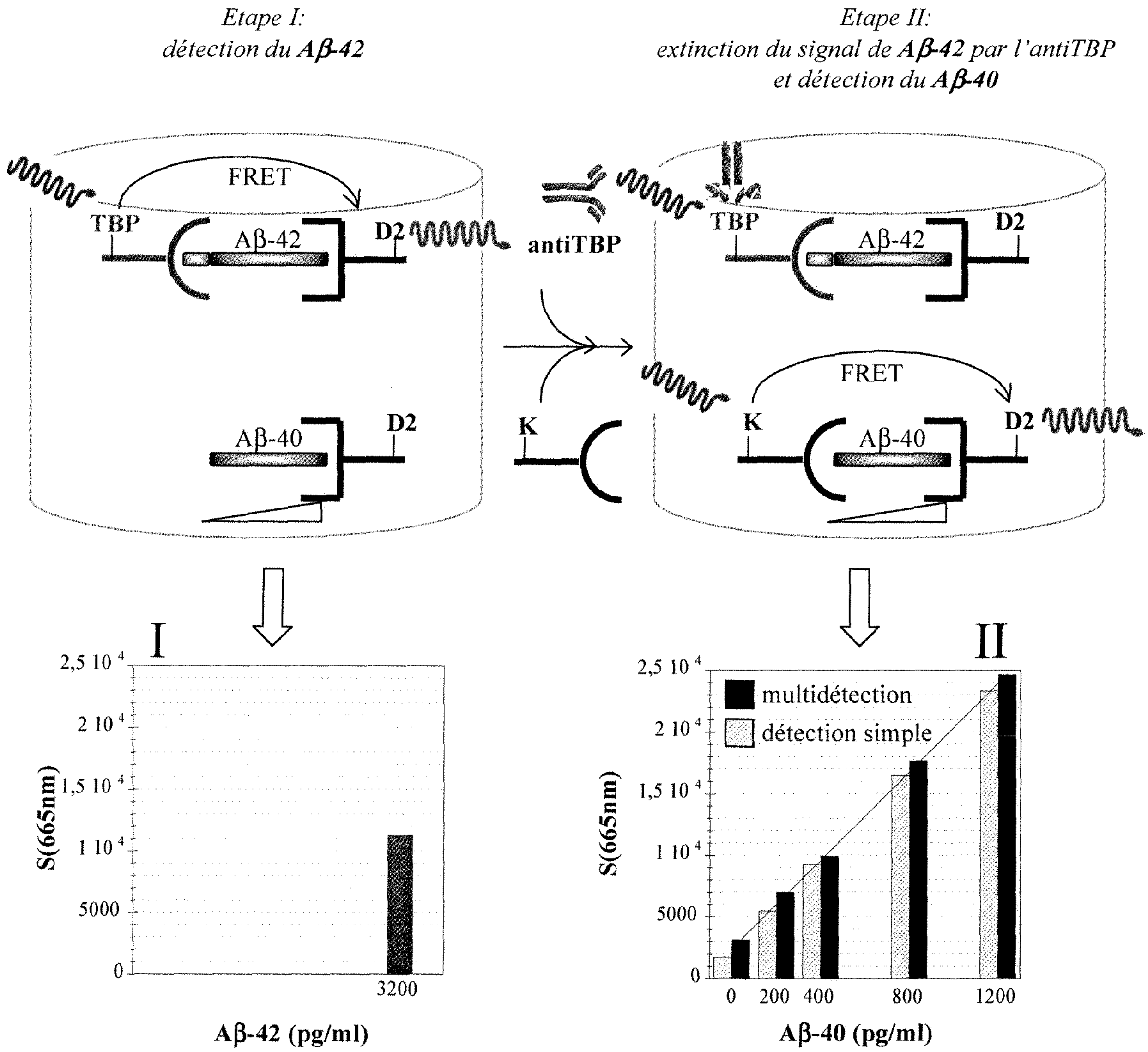

*Etape I:*
*détection du **Aβ-42***
*Etape II:*
*extinction du signal de **Aβ-42** par l'antiTBP*
*et détection du **Aβ-40***
FRET
TBP
Aβ-42
D2
antiTBP
Aβ-40
K
I
II
2,5 10 4
2 10 4
1,5 10 4
1 10 4
5000
0
S(665nm)
3200
Aβ-42 (pg/ml)
multidétection
détection simple
0
200
400
800
1200
Aβ-40 (pg/ml)


**Fig. 8**

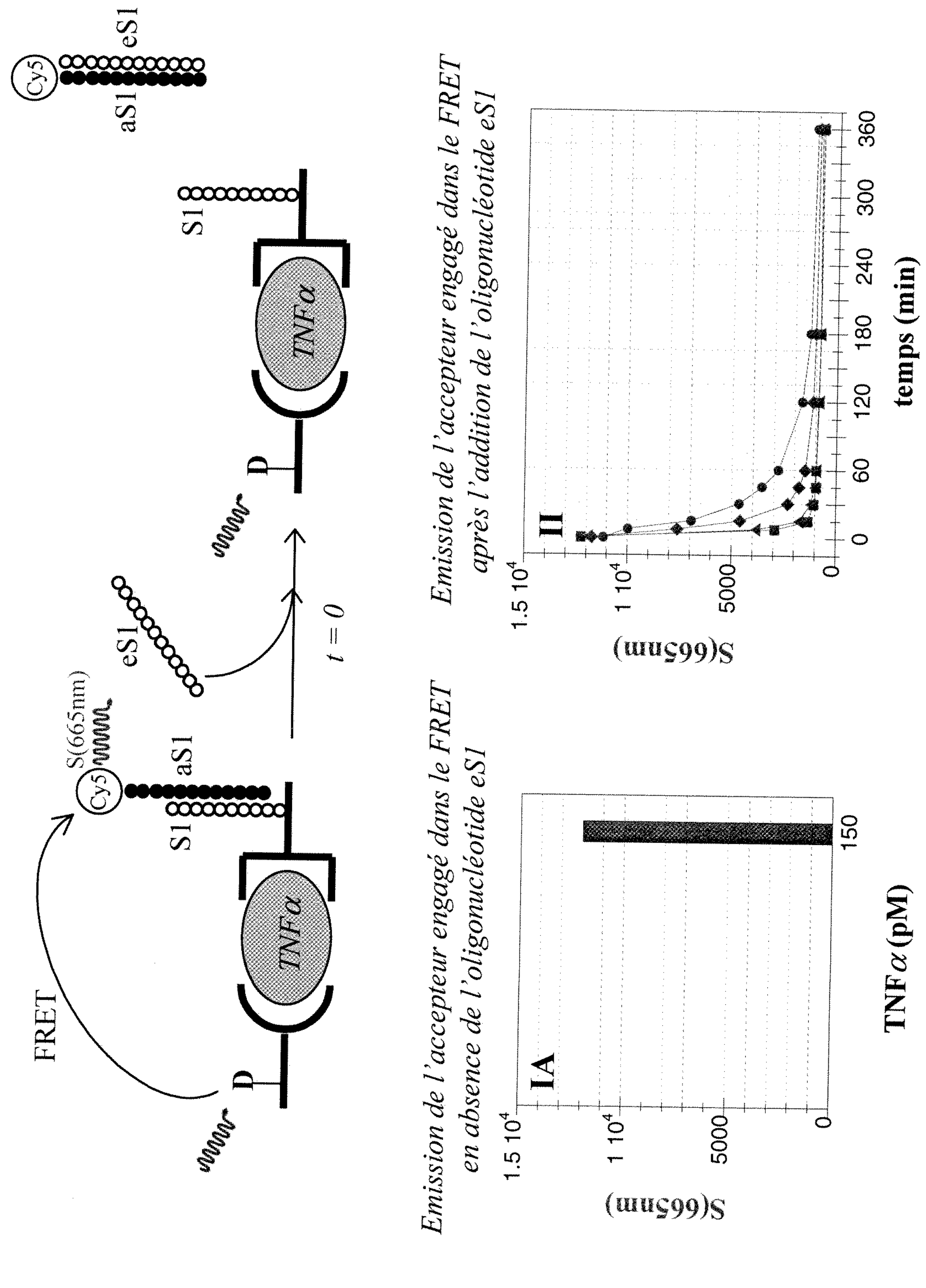
FRET
D
TNFα
S1
aS1
Cy5
S(665nm)
eS1
t = 0
Emission de l'accepteur engagé dans le FRET en absence de l'oligonucléotide eS1
IA
S(665nm)
1.5 10⁴
1 10⁴
5000
0
150
TNFα (pM)
Emission de l'accepteur engagé dans le FRET après l'addition de l'oligonucléotide eS1
II
0
60
120
180
240
300
360
temps (min)

Fig. 9

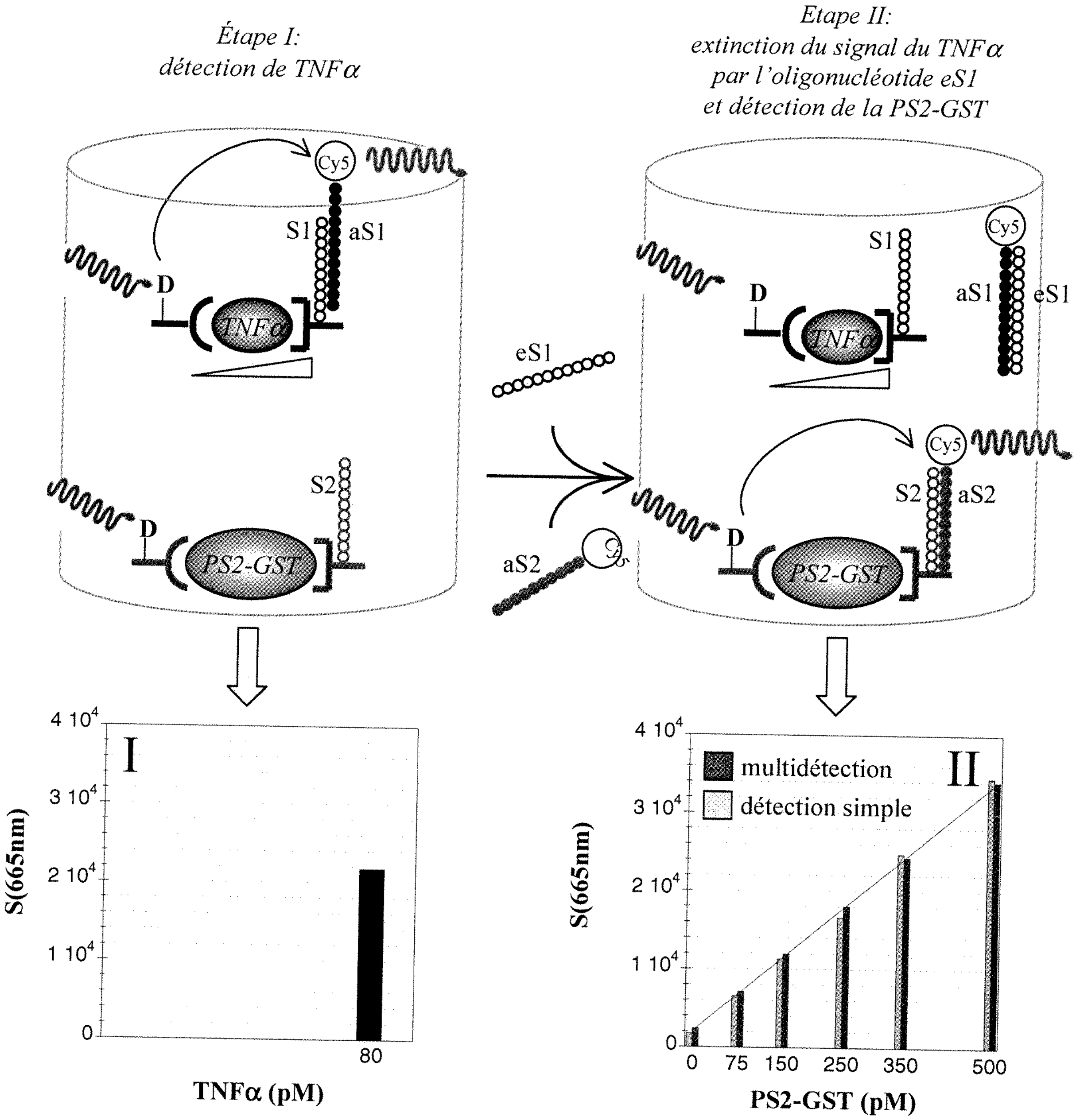
Étape I:
détection de TNFα
Etape II:
extinction du signal du TNFα
par l'oligonucléotide eS1
et détection de la PS2-GST
Cy5
S1
aS1
D
TNFα
S2
PS2-GST
eS1
aS2
I
II
4 10⁴
3 10⁴
2 10⁴
1 10⁴
0
S(665nm)
80
TNFα (pM)
multidétection
détection simple
0
75
150
250
350
500
PS2-GST (pM)

**Fig. 10**

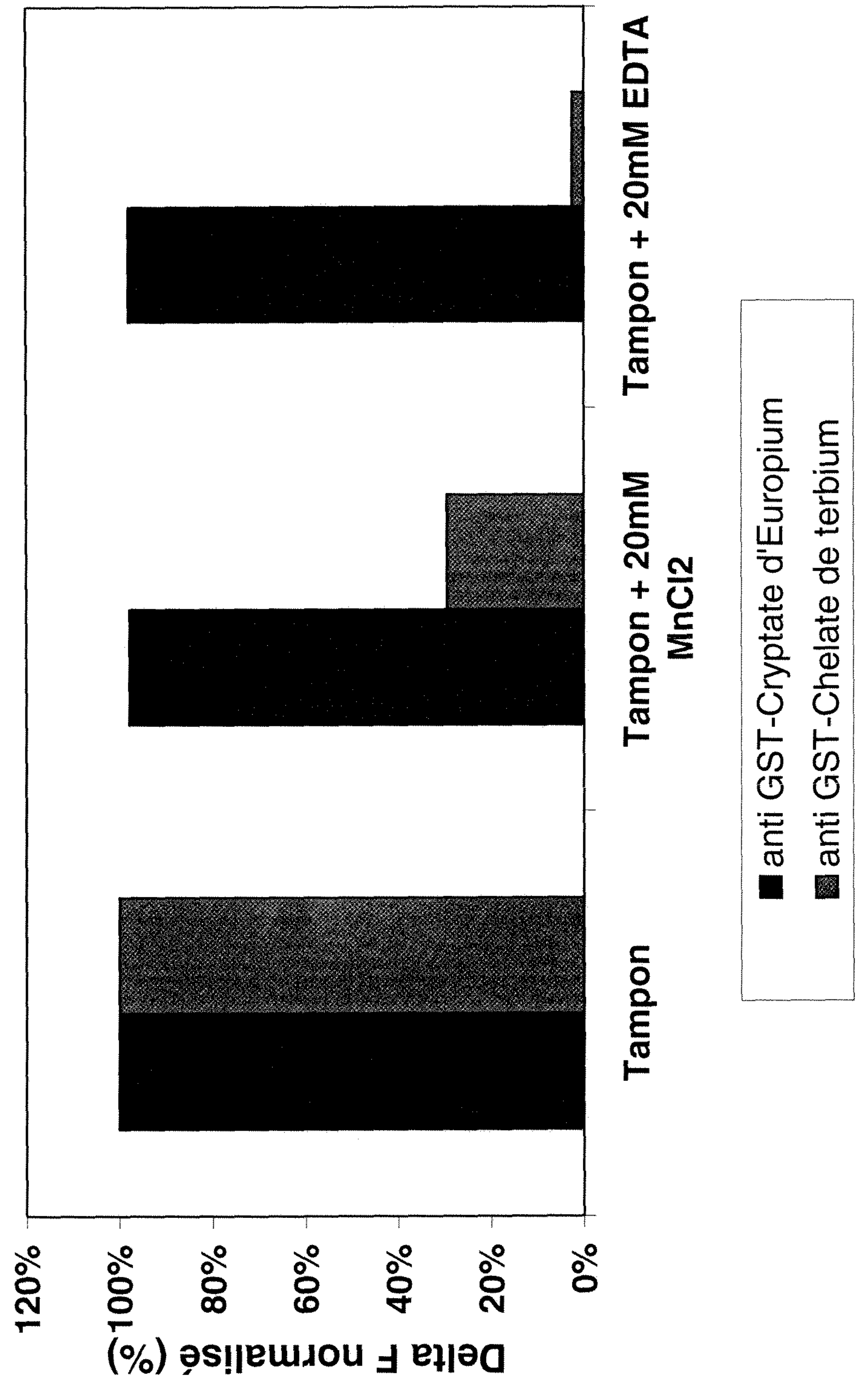
Delta F normalisé (%)
120%
100%
80%
60%
40%
20%
0%
Tampon
Tampon + 20mM MnCl2
Tampon + 20mM EDTA
anti GST-Cryptate d'Europium
anti GST-Chelate de terbium

Fig. 11

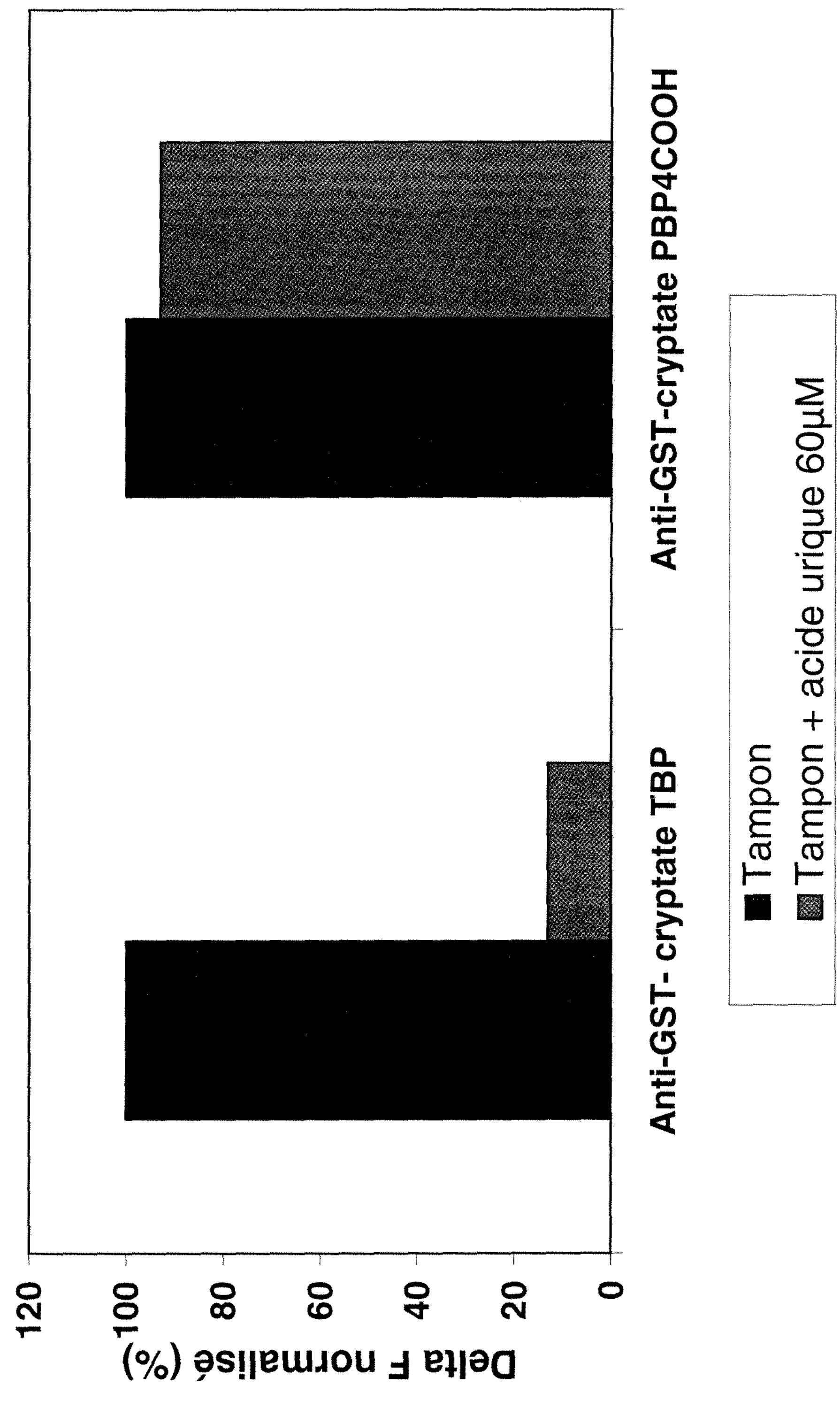
Delta F normalisé (%)
120
100
80
60
40
20
0
Anti-GST- cryptate TBP
Anti-GST-cryptate PBP4COOH
Tampon
Tampon + acide urique 60µM

Fig. 12

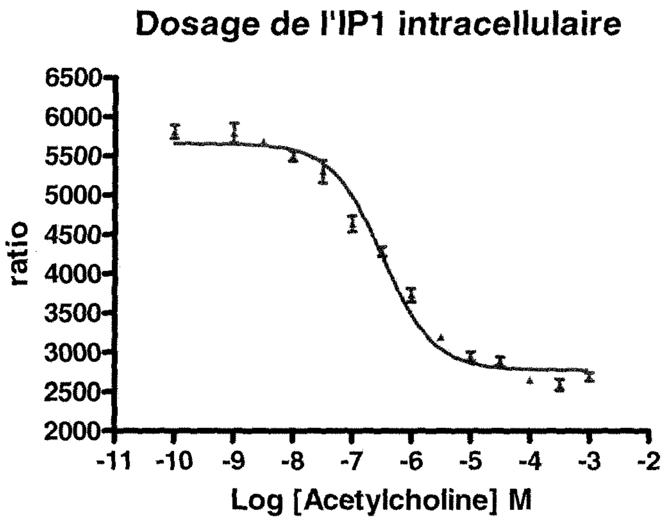
Dosage de l'IP1 intracellulaire
ratio
6500
6000
5500
5000
4500
4000
3500
3000
2500
2000
-11 -10 -9 -8 -7 -6 -5 -4 -3 -2
Log [Acetylcholine] M

**Fig. 13**

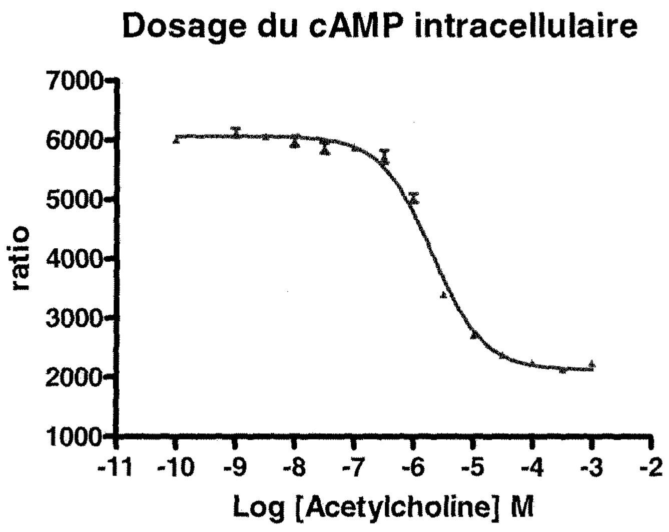
Dosage du cAMP intracellulaire
ratio
7000
6000
5000
4000
3000
2000
1000
-11 -10 -9 -8 -7 -6 -5 -4 -3 -2
Log [Acetylcholine] M

**Fig. 14**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6747135 B
- US 20050064512 A
- EP 321353 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 403593 A
- US 5324825 A
- US 5202423 A
- US 5316909 A
- EP 0180492 A
- EP 0601113 A
- WO 0196877 A
- EP 345315 A
- WO 9213264 A


### Littérature non-brevet citée dans la description


- **KIMURA et al.** *Forensic Sci Int,* 11 Septembre 2000, vol. 3, 345-51
- **LAKOWICZ.** Principles of fluorescence spectroscopy. Kluwer academic/plenum publishers, 1999
- **G.T. HERMANSON.** Bioconjugate Techniques. Academic Press, 1996
- **G. MATHIS.** *Clin. Chem.,* 1993, vol. 39, 1953
- **G. MATHIS.** *Clin. Chem.,* 1993, 1953
- **TRINQUET et al.** D-myo-inositol 1-phosphate as a surrogate of D-myo-inositol 1,4,5-tris phosphate to monitor G protein-coupled receptor activation. *Analytical Biochemistry,* 2006, vol. 358, 126-135
- **LYNCH BA ; MINOR C ; LOIACONO KA ; VAN SCHRAVENDIJK MR ; RAM MK ; SUNDARAMOORTHI R ; ADAMS SE ; PHILLIPS T ; HOLT D ; RICKLES RJ.** Simultaneous assay of Src SH3 and SH2 domain binding using different wavelength fluorescence polarization probes. *Anal Biochem.,* 1999, vol. 275 (1), 62-73
- **BLOMMEL P ; HANSON GT ; VOGEL KW.** Multiplexing fluorescence polarization assays to increase information content per screen: applications for screening steroid hormone receptors. *J Biomol Screen.,* 2004, vol. 9 (4), 294-302
- **SWARTZMAN et al.** An homogeneous and multiplexed immunoassay for high-throughput screening using fluorometric microvolume assay technology. *Anal. Biochem.,* 1999, vol. 271, 143-151
- **CHAN-HUI PY ; STEPHENS K ; WARNOCK RA ; SINGH S.** Applications of eTag trade mark assay platform to systems biology approaches in molecular oncology and toxicology studies. *Clin Immunol.,* 2004, vol. 111 (2), 162-74
- **TIAN H ; CAO L ; TAN Y ; WILLIAMS S ; CHEN L ; MATRAY T ; CHENNA A ; MOORE S ; HERNANDEZ V ; XIAO V.** Multiplex mRNA assay using electrophoretic tags for high-throughput gene expression analysis. *Nucleic Acids Res.,* 2004, vol. 32 (16), e126
- **BRADFORD JA ; BULLER G ; SUTER M ; IGNATIUS M ; BEECHEM JM.** Fluorescence-intensity multiplexing: simultaneous seven-marker, two-color immunophenotyping using flow cytometry. *ytometry A.,* 2004, vol. 61 (2), 142-52
- **KURNER JM ; KLIMANT I ; KRAUSE C ; PRINGSHEIM E ; WOLFBEIS OS.** A new type of phosphorescent nanospheres for use in advanced time-resolved multiplexed bioassays. *Anal Biochem.,* 2001, vol. 297 (1), 32-41
- **TONG AK ; LI Z ; JONES GS ; RUSSO JJ ; JU J.** Combinatorial fluorescence energy transfer tags for multiplex biological assays. *Nat Biotechnol.,* 2001, vol. 19 (8), 756-9
- **TYAGI S ; MARRAS SA ; KRAMER FR.** Wavelength-shifting molecular beacons. *Nat Biotechnol.,* 2000, vol. 18 (11), 1191-6
- **WATT et al.** *Immunochemistry,* 1997, vol. 14, 533-541
- **BABENDURE JR ; ADAMS SR ; TSIEN RY.** Aptamers switch on fluorescence of triphenylmethane dyes. *J Am Chem Soc.,* 2003, vol. 125 (48), 14716-7